# EUROPÄISCHE PATENTSCHRIFT

(11) **EP 3 317 410 B1**
(45) Veröffentlichungstag und Bekanntmachung des Hinweises auf die Patenterteilung: **25.08.2021**
(21) Anmeldenummer: 16736057.7
(22) Anmeldetag: 04.07.2016
(51) Int. Cl.: C12N 15/11, C12N 15/113, C12N 15/117, A61K 31/713, A61P 35/00, A61P 37/04

(54) **DISKONTINUIERLICHE OLIGONUKLEOTID-LIGANDEN**
DISCONTINUOUS OLIGONUCLEOTIDE LIGANDS
LIGANDS OLIGONUCLÉOTIDIQUES DISCONTINUS

(30) Priorität: 02.07.2015 DE 102015008536
(43) Veröffentlichungstag der Anmeldung: 09.05.2018
(73) Patentinhaber: Rheinische Friedrich-Wilhelms-Universität Bonn, 53113 Bonn (DE)
(72) Erfinder: GOLDECK, Marion, 1030 Wien (AT); LUDWIG, János, 37085 Göttingen (DE)
(74) Vertreter: Lahrtz, Fritz
(86) Internationale Anmeldenummer: PCT/EP2016/065686
(87) Internationale Veröffentlichungsnummer: WO 2017/001702

(56) Entgegenhaltungen:
- EP-A1- 2 407 539
- WO-A1-2008/049078
- WO-A1-2009/141146
- WO-A1-2011/031520
- WO-A2-2005/001055
- WO-A2-2007/117686
- LAN T ET AL: "Synthetic oligoribonucleotides-containing secondary structures act as agonists of Toll-like receptors 7 and 8", BIOCHEMICAL AND BIOPHYSICAL RESEARCH COMMUNICATIONS, ACADEMIC PRESS INC. ORLANDO, FL, US, Bd. 386, Nr. 3, 11. Juni 2009 (2009-06-11) , Seiten 443-448, XP026305579, ISSN: 0006-291X, DOI: 10.1016/J.BBRC.2009.06.036 [gefunden am 2009-06-11]
- ANNA AVIÑÓ ET AL: "Branched RNA: A New Architecture for RNA Interference", JOURNAL OF NUCLEIC ACIDS, Bd. 25, Nr. 23, 6. März 2011 (2011-03-06), Seite 586935, XP055301118, DOI: 10.1016/S0022-2836(03)00181-5
- SOO HYEON LEE ET AL: "Dual gene targeted multimeric siRNA for combinatorial gene silencing", BIOMATERIALS., Bd. 32, Nr. 9, 23. Dezember 2010 (2010-12-23), Seiten 2359-2368, XP055254453, GB ISSN: 0142-9612, DOI: 10.1016/j.biomaterials.2010.11.062
- HYUN JUNG CHUNG ET AL: "Reducible siRNA Dimeric Conjugates for Efficient Cellular Uptake and Gene Silencing", BIOCONJUGATE CHEMISTRY, Bd. 22, Nr. 2, 11. Januar 2011 (2011-01-11), Seiten 299-306, XP055099421, ISSN: 1043-1802, DOI: 10.1021/bc100438m
- YU DONG ET AL: "'Immunomers': Novel 3'-3'-linked CpG oligodeoxyribonucleotides as potent immunomodulatory agents", NUCLEIC ACIDS RESEARCH, INFORMATION RETRIEVAL LTD, GB, Bd. 30, Nr. 20, 15. Oktober 2002 (2002-10-15), Seiten 4460-4469, XP002447831, ISSN: 0305-1048, DOI: 10.1093/NAR/GKF582
- MASUDA H ET AL: "Synthesis, gene-silencing activity and nuclease resistance of 3'-3'-linked double short hairpin RNA", BIOORGANIC & MEDICINAL CHEMISTRY, PERGAMON, GB, Bd. 18, Nr. 23, 2. November 2010 (2010-11-02), Seiten 8277-8283, XP027546685, ISSN: 0968-0896 [gefunden am 2010-11-02]
- CINDY CHIANG ET AL: "Sequence-Specific Modifications Enhance the Broad-Spectrum Antiviral Response Activated by RIG-I Agonists", JOURNAL OF VIROLOGY, Bd. 89, Nr. 15, 27. Mai 2015 (2015-05-27), Seiten 8011-8025, XP055301314, US ISSN: 0022-538X, DOI: 10.1128/JVI.00845-15

## Beschreibung

Die vorliegende Erfindung betrifft ein Oligonukleotid-Konjugat K der Struktur
RNA1 -B- RNA2
RNA3 RNA4
oder ein pharmazeutisch aktives Salz davon, wobei RNA1, RNA2, RNA3 und RNA4 jeweils Stränge einer Ribonukleinsäure oder eines Analogons oder Derivats davon sind, wobei B einen bivalenten Linker darstellt, der den 5'-Terminus von RNA1 mit dem 5'-Terminus von RNA2 oder den 3'-Terminus von RNA1 mit dem 3'-Terminus von RNA2 kovalent verbindet, und wobei RNA3 und RNA4 nicht kovalent miteinander verbunden sind, wobei das Oligonukleotid-Konjugat K erfindungsgemäß ein Aktivator des zykllischen Helikase Retinsäure-Indizierbaren Gens I (RIG-I) ist,
wobei der bivalente Linker B erfindungsgemäß einen bivalenter Phosphodiester-Linker mit einem Molekulargewicht von nicht mehr als 1500 Da darstellt, und und wobei RNA1 und RNA3, sowie RNA2 und RNA4 erfindungsgemäß jeweils keinen Überhang der 5'-terminalen Nukleotidreste und nicht mehr als fünf Nukleotide Überhang der 3'-terminalen Nukleotidreste aufweisen, erfindungsgemäß zur Verwendung in einem Verfahren zum Behandeln oder Vorbeugen eines Tumors und/oder einer viralen Infektion. Ferner betrifft die vorliegende Erfindung die nichtmedizinische Verwendung eines solchen Oligonukleotid-Konjugats K und Herstellungsverfahren eines solchen.

Einige zytosolische Proteine, die in Schutzmechanismen für die Bekämpfung von Infektionen involviert sind, wie etwa zytosolischen Helikasen, können durch bestimmte doppelsträngige Ribonukleinsäuren (dsRNAs) aktiviert werden. Die Aktivierung solcher zytosolischen Proteine und Einleitung der entsprechenden Schutzmechanismen kann der Therapie von Neoplasien, Infektionen und/oder unkontrollierten Immunantworten dienen.

So kann etwa die zytosolischen Helikase RIG-I (Retinsäure-Induzierbares Gen I, engl. retinoic acid inducible gene I) durch dsRNAs aktiviert werden (Yoneyama et al., 2004, nat. Immunol. 5(7):730-737; Marques et al., 2006, Nat. Biotech. 24(5):559-565). Als noch wirksamer erwiesen sich solche RNAs mit 5'-terminalem Triphosphat (vgl. Hornung et al., 2006, Science 314:994-997; Pichelmair et al., 2006, Science 314:997-1001; Schmidt et al., 2009, PNAS 106(29):12067-12072; Schlee et al., 2009, Immunity 31:25-34). Die Stabilität und Bioaktivität dieser Moleküle ist jedoch noch unzureichend und verbesserungswürdig.

Grundsätzlich wurden zwar in anderen Zusammenhängen Ribonukleinsäure-(RNA-) Moleküle mit teilweise erhöhter Stabilität beschrieben, bei denen etwa zwei Einzelstränge jeweils über ihre 3'-Termini (3'-3'-Verknüpfung) verknüpft werden (vgl. US 2005/0026861, US 6,489,464, US 2008/0171712). Diese scheinen auch gewisse vorteilhafte Eigenschaften aufzuweisen. Diese lassen sich jedoch nicht ohne weiteres zur Aktivierung zytosolischer Proteine, die Schutzmechanismen für die Bekämpfung von Neoplasien, Injektionen und/oder unkontrollierter Immunantwort auslösen, wie etwa RIG-I, einsetzen. In der Regel handelt es sich hierbei um Einzelstränge, rückgefaltete Stränge, bei denen ein Teil beider kovalent verknüpfter RNA-Stränge miteinander hybridisiert, an einen gemeinsamen Strang hybridisierte RNAs oder polymere Strukturen aus 3'-3'-verknüpften Strukturen. Es ist jedoch nicht bekannt beide Stränge, die über eine 5'-5'-Verknüfung oder eine 3'-3'-Verknüfung miteinander verbunden sind, mit einem jeweils komplementären einzelnen, ungebundenen RNA-Strang zu hybridisieren.

WI 2007/117686 offenbart immunmodulatorische RNA-Konjugate, die multimere Strukturen ausbilden können. Auch EP-A 2407539 und Lee et al., 2011 (Biomaterials 32:2359-2368) lehren multimere RNA-Konjugate.

Lan et al., 2009 (Biochchemical and Biophysical Research Communications, 386:443-448) lehrt Toll-Like-Rezeptor-Agonisten, die verzweigte RNA-Oligomere enthalten. Auch Avino et al., 2011 (Journal of Nucleic Acids, Artikel ID 586935) lehrt verzweigte RNA-Oligomere.

Chung et al., 2011 (Bioconjugate Chemistry, 22:299-306) lehrt dimere Konjugate von siRNA, die grünfluoreszierendes Protein (GFP) oder vaskularen, endothelialen Wachstumsfaktor (VEGF) betrifft, wobei die RNA-Stränge über Alkyl-haltige Linker miteinander verknüpft sind. WO 2009/141146 lehrt doppelsträngige RNA-Konjugate, die über 3'-5'-Bindungen verknüpft sind, zur Behandlung von Tumoren.

Keine der aus dem Stand der Technik bekannten Strukturen ermöglicht eine Aktivierung von zellulären Faktoren wie etwa RIG-I im gewünschten Maße beziehungsweise ist zugleich synthetisch einfach zugänglich.

Daher war es erwünscht, Oligonukleotid-Konjugate mit verbesserten Eigenschaften zur Aktivierung von zellulären Faktoren wie etwa RIG-I bereitzustellen, die zudem auch noch synthetisch einfach zugänglich sind.

Überraschend wurde gefunden, dass ein Oligonukleotid-Konjugat K, bei dem mindestens zwei RNA-Stränge 5'-5'-oder 3'-3'-verknüpft sind, die mit weiteren RNA-Einzelsträngen zumindest teilweise hybridisiert sind, besonders vorteilhafte Eigenschaften in Bezug auf die Aktivierung von zytosolischen Proteinen, die in Schutzmechanismen für die Bekämpfung von Neoplasien, Injektionen und/oder unkontrollierter Immunantwort involviert sind, besonders zytosolische Helikasen, insbesondere RIG-I, hat.

Gelehrt wird hierin ein Oligonukleotid-Konjugat K der Struktur
RNA1 -B- RNA2
RNA3 RNA4
oder ein pharmazeutisch verträgliches Salz davon, wobei:
- RNA1: einen ersten Strang einer Ribonukleinsäure oder eines Analogons oder Derivats davon von wenigstens sechs Nukleotiden Länge darstellt;
- RNA2: einen zweiten Strang einer Ribonukleinsäure oder eines Analogons oder Derivats davon von wenigstens sechs Nukleotiden Länge darstellt;
- RNA3: einen dritten Strang einer Ribonukleinsäure oder eines Analogons oder Derivats davon von wenigstens sechs Nukleotiden Länge darstellt, der mindestens fünf komplementäre Basenpaarungen zu RNA1 ausbildet;
- RNA4: einen vierten Strang einer Ribonukleinsäure oder eines Analogons oder Derivats davon von wenigstens sechs Nukleotiden Länge darstellt, der mindestens fünf komplementäre Basenpaarungen zu RNA2 ausbildet; und
- B: einen bivalenten Linker darstellt, der den 5'-Terminus von RNA1 mit dem 5'-Terminus von RNA2 oder den 3'-Terminus von RNA1 mit dem 3'-Terminus von RNA2 kovalent verbindet,
wobei RNA3 und RNA4 nicht kovalent miteinander verbunden sind.

In anderen Worten ausgedrückt verbindet der bivalente Linker B RNA1 und RNA2 so miteinander, dass eine 5'-5' Verknüpfung oder eine weitere 3'-3'-Verknüpfung im beschriebenen Oligonukleotid-Konjugat K vorliegt. Bevorzugt liegt im gesamten Oligonukleotid-Konjugat K nur eine einzige 5'-5'-Verknüpfung oder 3'-3'-Verknüpfung vor.

Die vorliegende Erfindung betrifft ein solches Oligonukleotid-Konjugat K, das ein Aktivator des zykllischen Helikase Retinsäure-Indizierbaren Gens I (RIG-I) ist,
wobei der bivalente Linker B einen bivalenter Phosphodiester-Linker mit einem Molekulargewicht von nicht mehr als 1500 Da darstellt, und
wobei RNA1 und RNA3, sowie RNA2 und RNA4 jeweils keinen Überhang der 5'-terminalen Nukleotidreste und nicht mehr als fünf Nukleotide Überhang der 3'-terminalen Nukleotidreste aufweisen,
zur Verwendung in einem Verfahren zum Behandeln oder Vorbeugen eines Tumors und/oder einer viralen Infektion.

Bevorzugt besteht das Oligonukleotid-Konjugat K oder ein pharmazeutisch verträgliches Salz davon aus der Struktur
RNA1 -B- RNA2
RNA3 RNA4

Dem Fachmann wird ersichtlich sein, dass eine Ribonukleinsäure, ein Analogon oder Derivat davon auch die jeweiligen pharmazeutisch verträglichen Salze davon einschließen, die sich in wässriger Umgebung, abhängig vom pH-Wert, bilden oder bilden können.

Beispiele für Gegenkationen sind etwa H⁺, Na⁺, K⁺, Mg²⁺, Ca²⁺, Zn²⁺ sowie die Kationen von Aminosäuren (z.B. Lysin, Arginin, Prolin) und Cholin.

RNA1, RNA2, RNA3 und RNA4 stellen jeweils einen durchgängigen zusammenhängenden Strang dar. RNA1, RNA2, RNA3 und RNA4 weisen daher jeweils einen 3'- und einen 5'-Terminus auf. Daher tritt in RNA1, RNA2, RNA3 und RNA4 keine Unterbrechung der Sequenz dahingehend ein, dass innerhalb dieser Stränge eine weitere 5'-5'-Verküpfung oder eine weitere 3'-3'-Verknüpfung vorkommt.

Ausführlicher dargestellt liegt eine der folgenden Strukturen vor:
Beschriebenes Oligonukleotid-Konjugat K mit 5'-5'-Verknüpfung:
3'-RNA1-5' -B- 5'-RNA2-3'
5'-RNA3-3' 3'-RNA4-5'

Beschriebenes Oligonukleotid-Konjugat K mit 3'-3'-Verknüpfung
5'-RNA1-3' -B- 3'-RNA2-5'
3'-RNA3-5' 5'-RNA4-3'

Ein Analogon einer Ribonukleinsäure (RNA-Analogon) kann ein beliebiges im Stand der Technik bekanntes RNA-Analogon sein. Beispielsweise kann ein RNA-Analogon ausgewählt sein aus der Gruppe bestehend aus Peptidnukleinsäure (engl. peptide nucleic acid, PNA), Morpholinos, Glykol-Nukleinsäure (engl. glycol nucleic acid, GNA), Threose-Nukleinsäure (engl. threose nucleic acid, TNA), verriegelte Nucleoside (engl. locked nucleosides, LNA) und 2',3'-Seco-Nucleoside (UNA). Ferner können die Oligonukleotide nicht nukleotidische Bausteine und/oder Seitenketten-Modifikationen umfassen. Beispielsweise kann die 2'-OH einer Ribonukleosid-Untereinheit durch eine Gruppe, ausgewählt aus OR, R, Halogen, SH, SR, NH₂, NHR, NR₂ oder CN ersetzt sein, wobei R ein optional substituiertes C₁₋₆-Alkyl, C₂₋₆-Alkenyl, C₂₋₆-Alkinyl und Halogen bevorzugt für F, Cl, Br oder I steht. Ferner kann die Ribose durch einen anderen Zucker wie zum Beispiel eine andere Pentose (z.B. Arabinose) ersetzt sein. Die Verknüpfung durch zwei Zucker kann auch über eine 2'-fluoroarabinonucleoside Untereinheiten erfolgen. Ebenso können Phosphorothioate, Phosphorodithioate, Methylphosphonate, Phosphoramidate, Boranophosphate oder andere modifizierte Bindungen verwendet werden.

Ein Derivat einer Ribonukleinsäure (RNA-Derivat) kann eine beliebige abgewandelte, chemisch oder biochemisch veränderte Form einer Ribonukleinsäure sein. Beispielsweise kann ein RNA-Derivat methylierte RNA sein. Beispielsweise kann eine Base oder können zwei oder mehr Basen oder können alle Basen alkyliert (z.B. methyliert) und/oder halogeniert (z.B. fluoriert) sein. Auch können einer oder mehrere der Zuckerreste (Ribosereste) der Nukleinsäure einfach oder mehrfach modifiziert sein. So kann beispielsweise ein Riboserest alkyliert (z.B. methyliert) sein. Bevorzugt kann ein Riboserest 2'-O-alkyliert (z.B. 2'-O-methyliert) sein. Alternativ oder zusätzlich kann ein oder können mehrere Phosphothioate als Verbindungen zwischen den Nukleotiden vorliegen.

Es können auch Nicht-Standard-Nukleobasen verwendet werde wie beispielsweise 5-(2-Amino)propyl-Uracil, 5-Bromuracil, Hypoxanthin; 2,6-Diaminopurin; 8-Bromguanin, 7-Desazaguanin, 7-Deazaadenin; N⁶-Methyladenin, N⁶-Dimethyladenin oder 5-Nitroindol.

Weitere im Zusammenhang mit der vorliegenden Erfindung einsetzbare Modifikationen sind in WO 2014/049079 gezeigt. Beispiele für RNA-Derivate sind im Beispielteil der vorliegenden Anmeldung gezeigt.

Bevorzugt wird die Aktivität der entsprechend modifizierten Ribonukleinsäure nicht zu stark eingeschränkt. So bleibt bevorzugt mindestens 60%, noch stärker bevorzugt mindestens 70%, noch stärker bevorzugt mindestens 80%, insbesondere bevorzugt mindestens 90% der Aktivität (z.B. der RIG-I-aktivierenden Aktivität) erhalten im Vergleich zu der entsprechenden unmodifizierten RNA. Es kann sogar zu einer Aktivitätssteigerung kommen, die besonders bevorzugt ist.

Bevorzugt erhöhen die Modifikationen die biologische Stabilität und Lagerstabilität des Nukleinsäure-Stranges und/oder des gesamten Oligonukleotid-Konjugats K gemäß der vorliegenden Erfindung.

Der Fachmann wird erkennen, dass nicht zwangsläufig alle Nukleotide eines Strangs einheitlich allesamt Ribonukleinsäure-Nukleotide, RNA-Analogon-Nukleotide oder RNA-Derivat-Nukleotide sind. Vielmehr können auch einzelne Nukleotide RNA-Analogon-Nukleotide und/oder RNA-Derivat-Nukleotide sein und der Rest des Strangs (weitgehend) aus Ribonukleinsäure-Nukleotiden bestehen oder umgekehrt.

Das beschriebene Oligonukleotid-Konjugat K kann ein beliebiges Ziel ansprechen.

Erfindungsgemäß ist das Oligonukleotid-Konjugat K ein Aktivator der zytosolischen Helikase Retinsäure-Induzierbares Gen I (RIG-I) zur Verwendung in einem Verfahren zum Behandeln oder Vorbeugen eines Tumors und/oder einer viralen Infektion.

Bevorzugt weist RIG-I wie hierin verwendet mindestens 80% Homologie zu Gen-Name DDX58 beziehungsweise UniProt ID O95786 beziehungsweise OMIM ID 609631 auf. Es handelt sich um eine Helikase. Auch kann RIG-I als Probable ATP-dependent RNA helicase (DDX58) bezeichnet werden. Stärker bevorzugt weist RIG-I wie hierin verwendet mindestens 90%, noch stärker bevorzugt mindestens 95%, noch stärker bevorzugt mindestens 98% Homologie, insbesondere Identität zu Gen-Name DDX58 auf.

Es handelt es sich bei zytosolischen Helikase Retinsäure-Induzierbares Gen I (RIG-I, engl. retinoic acid inducible gene I) um eine zytosolische Helikase, die als Nukleinsäurerezeptor des angeborenen Immunsystems dient. Die Aktivierung von RIG-I führt üblicherweise zu der Auslösung einer Signalkaskade, die zur Ausschüttung von zahlreichen Zytokinen und Chemokinen, insbesondere von Typ-I Interferonen (z.B. Interferon alpha (INFα)) führt und angeborene und adaptive Immunantworten fördert.

Eine Endstruktur der Dimereinheit RNA1/RNA3 und/oder der Dimereinheit RNA2/RNA4 kann komplementär zu einer Domäne des Zielproteins, etwa einer C-terminalen Domäne, sein, beispielsweise zu der C-terminalen Domäne (CTD) des RIG-I-Proteins. Dies kann insbesondere dann erfüllt sein, wenn das dem Linker B entgegengesetzte 5'-Ende der Dimereinheit RNA1/RNA3 und/oder der Dimereinheit RNA2/RNA4 einen Doppelstrang mit stumpfem Ende (engl. blunt end) bildet. Die hier beschriebenen Oligonukleotid-Konjugate K sind gemäß einer besonders bevorzugten Ausführungsform dadurch gekennzeichnet dass sie zwei identische CTD-bindende Stränge ohne Überhänge (engl. blunt end) in symmetrischer Anordnung aufweisen, was vorteilhaft für die Wechselwirkung mit RIG-I sein kann.

Bevorzugt ist ein Aktivator der RIG-I dadurch gekennzeichnet, dass die Sekretion von Interferon alpha (IFNα) durch die Zellen, die mit einem beschriebenen Oligonukleotid-Konjugat K behandelt werden, höher ist als bei entsprechenden Zellen, die nicht mit einem beschriebenen Oligonukleotid-Konjugat K behandelt werden. Bei den Zellen handelt es sich hierbei bevorzugt um mononukleäre Zellen des peripheren Blutes (engl. Peripheral Blood Mononuclear Cells, PBMCs), die besonders bevorzugt mit üblichen Dosen Chloroquin vorbehandelt sind.

Stärker bevorzugt steigt bei einer Zugabe von 5 nM des beschriebenen Oligonukleotid-Konjugats K die IFNα-Sekretion von Chloroquin-behandelten PBMCs um mindestens 50%, bevorzugt mindestens das 2fache, stärker bevorzugt mindestens das 5fache, insbesondere mindestens das 10fache oder gar mehr als das 20fache im Vergleich zu entsprechenden Chloroquin-behandelten PBMCs ohne Zugabe des Oligonukleotid-Konjugats K. Dies ist auch durch die unten gezeigten Beispiele weiter verdeutlicht.

Die Sequenzen RNA1 und RNA2 können verschieden sein, können einander ähnlich sein oder können einander identisch sein. So können die Sequenzen von RNA1 und RNA2 eine Sequenzhomologie von unter 60%, von mindestens 60%, von mindestens 70%, von mindestens 80%, von mindestens 90%, von mindestens 95%, von mindestens 98% oder von 100% (daher vollständiger Identität) aufweisen. Bevorzugt sind RNA1 und RNA2 einander zumindest ähnlich, weisen stärker bevorzugt mindestens 60%, noch stärker bevorzugt mindestens 70%, noch stärker bevorzugt mindestens 80%, noch stärker bevorzugt mindestens 90%, noch stärker bevorzugt mindestens 95%, noch stärker bevorzugt mindestens 98%, insbesondere über 98% Sequenzhomologie auf.

Gemäß einer besonders bevorzugten Ausführungsform ist die Sequenz von RNA2 der Sequenz von RNA1 identisch.

Ebenso können die Sequenzen RNA3 und RNA4 verschieden sein, können einander ähnlich sein oder können identisch sein. So können die Sequenzen von RNA3 und RNA4 eine Sequenzhomologie von unter 60%, von mindestens 60%, von mindestens 70%, von mindestens 80%, von mindestens 90%, von mindestens 95%, von mindestens 98% oder von 100% (daher vollständiger Identität) aufweisen. Bevorzugt sind RNA3 und RNA4 einander zumindest ähnlich, weisen stärker bevorzugt mindestens 60%, noch stärker bevorzugt mindestens 70%, noch stärker bevorzugt mindestens 80%, noch stärker bevorzugt mindestens 90%, noch stärker bevorzugt mindestens 95%, noch stärker bevorzugt mindestens 98%, insbesondere über 98% Sequenzhomologie auf.

Gemäß einer besonders bevorzugten Ausführungsform ist die Sequenz von RNA3 der Sequenz von RNA4 identisch.

Wie aus der oben dargestellten Struktur ersichtlich und im Text definiert, sind RNA3 zu RNA1 sowie RNA4 zu RNA2 zumindest teilweise komplementär. Daher sind noch stärker bevorzugt RNA1 zu RNA2 sowie RNA3 zu RNA4 einander jeweils zumindest ähnlich, weisen stärker bevorzugt jeweils mindestens 60%, noch stärker bevorzugt jeweils mindestens 70%, noch stärker bevorzugt jeweils mindestens 80%, noch stärker bevorzugt jeweils mindestens 90%, noch stärker bevorzugt jeweils mindestens 95%, noch stärker bevorzugt jeweils mindestens 98%, insbesondere jeweils über 98% Sequenzhomologie auf.

Am stärksten bevorzugt ist die Sequenz von RNA2 der Sequenz von RNA1 identisch und die Sequenz von RNA3 der Sequenz von RNA4 identisch.

In diesem Falle ist das Oligonukleotid-Konjugat K (weitgehend) symmetrisch. Wie oben dargestellt, stellt B in der oben gezeigten Struktur einen bivalenten Linker dar, der den 5'-Terminus von RNA1 mit dem 5'-Terminus von RNA2 oder den 3'-Terminus von RNA1 mit dem 3'-Terminus von RNA2 kovalent verbindet. Bevorzugt stellt B einen bivalenten Linker dar, der den 5'-Terminus von RNA1 mit dem 5'-Terminus von RNA2 kovalent verbindet. Daher sind RNA1 und RNA2 bevorzugt über ihre 5'-Termini jeweils kovalent an den bivalenten Linker B gebunden.

Die 5'-Termini von RNA3 und RNA4 können Triphosphatreste, Triphosphatanalogonreste, freie Hydroxylgruppen oder andere Reste aufweisen, an feste Träger oder polymere Strukturen gebunden sein. Bevorzugt weisen die 5'-Termini von RNA3 und RNA4 jeweils Triphosphatreste, Triphosphatanalogonreste oder freie Hydroxylgruppen auf. Stärker bevorzugt weisen die 5'-Termini von RNA3 und RNA4 jeweils Triphosphatreste oder freie Hydroxylgruppen auf. Noch stärker bevorzugt weisen die 5'-Termini von RNA3 und RNA4 jeweils freie Hydroxylgruppen auf.

Gemäß einer stark bevorzugten Ausführungsform stellt B einen bivalenten Linker dar, der den 5'-Terminus von RNA1 mit dem 5'-Terminus von RNA2 kovalent verbindet, und weisen RNA3 und RNA4 an ihren 5'-Termini jeweils Triphosphatreste, Triphosphatanalogonreste oder freie Hydroxylgruppen auf.

Gemäß einer stärker bevorzugten Ausführungsform stellt B einen bivalenten Linker dar, der den 5'-Terminus von RNA1 mit dem 5'-Terminus von RNA2 kovalent verbindet, und weisen RNA3 und RNA4 an ihren 5'-Termini jeweils Triphosphatreste oder freie Hydroxylgruppen auf.

Gemäß einer noch stärker bevorzugten Ausführungsform stellt B einen bivalenten Linker dar, der den 5'-Terminus von RNA1 mit dem 5'-Terminus von RNA2 kovalent verbindet, und weisen RNA3 und RNA4 an ihren 5'-Termini jeweils Triphosphatreste auf.

Dann ergibt sich ganz besonders bevorzugt folgende Struktur für das Oligonukleotid-Konjugat K mit 5'-5'-Verknüpfung und Triphosphatresten (ppp):
3'-RNA1-5' -B- 5'-RNA2-3'
5'-ppp-RNA3-3' 3'-RNA4-ppp-5'

Alternativ können die 3'-Termini von RNA1 und RNA2 jeweils an den bivalenten Linker B gebunden sein. Dann können die 5'-Termini von RNA1 und RNA2 Triphosphatreste, Triphosphatanalogonreste, freie Hydroxylgruppen oder andere Reste aufweisen, an feste Träger oder polymere Strukturen gebunden sein.

Gemäß einer bevorzugten Ausführungsform stellt B dann einen bivalenten Linker-Rest dar, der den 3'-Terminus von RNA1 mit dem 3'-Terminus von RNA2 kovalent verbindet, und RNA1 und RNA2 weisen an ihren 5'-Termini jeweils Triphosphatreste, Triphosphatanalogonreste oder freie Hydroxylgruppen auf.

Wenn der bivalenten Linker-Rest Phosphat ist, ist das erste 3'-terminale Nukleotid bevorzugt substituiert (z.B. 2'-substituiert (z.B. 2'-methyliert)). Dies kann die chemische Stabilität erhöhen.

Gemäß einer stärker bevorzugten Ausführungsform stellt B dann einen bivalenten Linker-Rest dar, der den 3'-Terminus von RNA1 mit dem 3'-Terminus von RNA2 kovalent verbindet, und RNA1 und RNA2 weisen an ihren 5'-Termini jeweils Triphosphatreste oder freie Hydroxylgruppen auf.

Gemäß einer noch stärker bevorzugten Ausführungsform stellt B dann einen bivalenten Linker-Rest dar, der den 3'-Terminus von RNA1 mit dem 3'-Terminus von RNA2 kovalent verbindet, und RNA1 und RNA2 weisen an ihren 5'-Termini jeweils Triphosphatreste auf.

Wie oben dargelegt, weisen die Sequenzen RNA1, RNA2, RNA3 und RNA4 jeweils mindestens sechs Nukleotide Länge auf.

Gemäß einer bevorzugten Ausführungsform weisen RNA1, RNA2, RNA3 und RNA4 jeweils zwischen 10 und 50, bevorzugt zwischen 15 und 40, stärker bevorzugt zwischen 19 und 30, insbesondere zwischen 20 und 25 Nukleotide Länge auf.

Beispielsweise kann die Länge an Nukleotiden jeweils unabhängig voneinander 19, 20, 21, 22, 23, 24, 25, 26, 27, 28 oder 29 Nukleotide betragen.

Bevorzugt weist das beschriebene Oligonukleotid-Konjugat K durch RNA1/RNA3 und RNA2/RNA4 gebildete Doppelstränge von jeweils mindestens 19 Basenpaaren (bp) Länge auf. So können die durch RNA1/RNA3 und RNA2/RNA4 gebildeten Doppelstränge beispielsweise unabhängig voneinander eine Länge von 19, 20, 21, 22, 23, 24, 25, 26, 27, 28 oder 29 bp aufweisen.

In dem Oligonukleotid-Konjugat K können RNA1 und RNA3, sowie RNA2 und RNA4 jeweils einen Überhang der 5'-terminalen Nukleotidreste aufweisen oder keinen Überhang der 5'-terminalen Nukleotidreste aufweisen. Erfindungsgemäß weisen RNA1 und RNA3, sowie RNA2 und RNA4 jeweils keinen Überhang der 5'-terminalen Nukleotidreste auf.

Ferner können in dem Oligonukleotid-Konjugat K RNA1 und RNA3, sowie RNA2 und RNA4 jeweils einen Überhang der 3'-terminalen Nukleotidreste aufweisen oder keinen Überhang der 3'-terminalen Nukleotidreste aufweisen. Erfindungsgemäß weisen RNA1 und RNA3, sowie RNA2 und RNA4 jeweils nicht mehr als fünf, bevorzugt nicht mehr als vier, stärker bevorzugt nicht mehr als drei, noch stärker bevorzugt nicht mehr als zwei Nukleotide, noch stärker bevorzugt nicht mehr als ein Nukleotid Überhang der 5'-terminalen Nukleotidreste auf. Besonders bevorzugt weisen RNA1 und RNA3, sowie RNA2 und RNA4 jeweils keinen Überhang der 5'-terminalen Nukleotidreste auf.

Daher weisen gemäß einer bevorzugten Ausführungsform RNA1 und RNA3, sowie RNA2 und RNA4 jeweils keinen Überhang der 5'-terminalen Nukleotidreste und nicht mehr als fünf, bevorzugt nicht mehr als vier, stärker bevorzugt nicht mehr als drei, noch stärker bevorzugt nicht mehr als zwei, noch stärker bevorzugt nicht mehr als ein Nukleotid Überhang der 3'-terminalen Nukleotidreste auf.

Gemäß einer stärker bevorzugten Ausführungsform weisen RNA1 und RNA3, sowie RNA2 und RNA4 jeweils keinen Überhang der 5'-terminalen Nukleotidreste und keinen Unterschied in der Länge des Nukleotidstrangs auf.

Gemäß einer ganz besonders bevorzugten Ausführungsform ist RNA1 zu RNA3 vollständig komplementär und RNA2 zu RNA4 vollständig komplementär.

Daher weist dann RNA1 die gleiche Länge auf wie RNA3 und RNA2 weist die gleiche Länge auf wie RNA4 und alle Basen gehen bevorzugt entsprechende Basenpaarungen ein. Es gibt dann weder eine 5'- noch einen 3'-Überhang, sondern ein stumpfes Ende (engl. blunt end)

Da wie oben dargelegt auch RNA1 und RNA2 bevorzugt zumindest ähnlich, insbesondere (weitgehend) identisch sind, sind besonders bevorzugt die Stränge RNA1, RNA2, RNA3, RNA4 jeweils gleich lang.

Gemäß einer besonders bevorzugten Ausführungsform ist daher die Sequenz von RNA2 der Sequenz von RNA1 identisch und die Sequenz von RNA3 der Sequenz von RNA4 identisch,
RNA1 zu RNA3 vollständig komplementär,
RNA2 zu RNA4 vollständig komplementär, und
sind die Stränge RNA1, RNA2, RNA3, RNA4 jeweils gleich lang.

Erfindungsgemäß ist der bivalente Linker B in dem beschriebenen Oligonukleotid-Konjugat K ein Phosphodiester-Linker. Erfindungsgemäß weist der bivalente Linker B dabei ein Molekulargewicht von nicht mehr als 1500 Da auf.

Es wird gelehrt, dass der bivalente Linker B ausgewählt sein kann aus der Gruppe bestehend aus einem Phosphodiester-Linker (erfindungsgemäß) oder einem Analogon oder Derivat davon, einem Glykollinker, einem Polyethylenglycol-Linker, einem Kohlenhydrat-Linker, einem Ribonukleinsäure-Linker, einem Desoxyribonukleinsäure-Linker, einem unsubstituierten oder substituierten C₁-C₁₂-Alkyl-Linker, einem Aminosäure-Linker, einem Peptid-Linker, und Kombinationen zweier oder mehr daraus, wobei der Linker ein Molekulargewicht von nicht mehr als 1500 Da hat.

Stärker bevorzugt weist der bivalente Linker B ein Molekulargeweicht von nicht mehr als 1000 Da auf, insbesondere ein Molekulargewicht von nicht mehr als 500 Da. Beispiele für bivalente Linker B sind im Beispielteil unten verdeutlicht.

Gemäß einer besonders bevorzugten Ausführungsform ist der bivalente Linker B ein Phosphodiesterlinker:
RNA1-O-PO(OH)-O-RNA2
RNA3 RNA3
beziehungsweise ein pharmazeutisch verträgliches Salz davon:
RNA1-O-PO(O⁻X⁺)-O-RNA2,
RNA3 RNA3
wobei X⁺ ein pharmazeutisch verträgliches Kation ist, wie beispielsweise H⁺, Na⁺, K⁺, Ca²⁺, Mg²⁺ usw.

RNA1, RNA2, RNA3 und RNA4 können jeweils unabhängig von einander eine beliebige Sequenz aufweisen.

Bevorzugt weist mindestens einer der Stränge ausgewählt aus RNA1, RNA2, RNA3 und RNA4 eine Sequenzhomologie zu SEQ ID NO: 1 oder SEQ ID NO: 2 von mindestens 80% auf. Diese Sequenzen sind bevorzugt Einzelstränge und bilden nicht oder kaum intramolekulare Tertiärstrukturen aus, was die spätere Hybridisierung mit den jeweils komplementären Strängen erleichtert. Die Sequenzen sind unten dargestellt. Stärker bevorzugt weisen mindestens zwei der Stränge ausgewählt aus RNA1, RNA2, RNA3 und RNA4 eine Sequenzhomologie zu SEQ ID NO: 1 oder SEQ ID NO: 2 von mindestens 80% auf. Noch stärker bevorzugt weisen zwei der Stränge ausgewählt aus RNA1, RNA2, RNA3 und RNA4 eine Sequenzhomologie zu SEQ ID NO: 1 von mindestens 80% und die zwei anderen eine Sequenzhomologie zu SEQ ID NO: 2 von mindestens 80% auf. Hierbei weisen RNA1 und RNA3 nicht die gleichen Sequenzen, sondern bevorzugt die jeweils andere Sequenz auf, sowie RNA2 und RNA4 nicht die gleichen Sequenzen, sondern bevorzugt die jeweils andere Sequenz auf.

Gemäß einer stärker bevorzugten Ausführungsform weist RNA1 oder RNA3 eine Sequenzhomologie zu SEQ ID NO: 1 von mindestens 80% auf.

Gemäß einer stärker bevorzugten Ausführungsform weist RNA1 eine Sequenzhomologie zu SEQ ID NO: 1 von mindestens 80% auf:
5'-GACGCUGACCCUGAAGUUCAUCUU-3' (SEQ ID NO: 1)

Stärker bevorzugt weist RNA1 eine Sequenzhomologie zu SEQ ID NO: 1 von mindestens 90%, noch stärker bevorzugt von mindestens 95%, noch stärker bevorzugt von mindestens 98% auf. Insbesondere weist RNA1 die SEQ ID NO: 1 auf.

Gemäß einer noch stärker bevorzugten Ausführungsform weisen RNA1 und RNA2 oder RNA3 und RNA4 jeweils eine Sequenzhomologie zu SEQ ID NO: 1 von mindestens 80% auf.

Gemäß einer noch stärker bevorzugten Ausführungsform weisen RNA1 und RNA2 jeweils eine Sequenzhomologie zu SEQ ID NO: 1 von mindestens 80% auf.

Stärker bevorzugt weisen RNA1 und RNA2 oder RNA3 und RNA4 jeweils eine Sequenzhomologie zu SEQ ID NO: 1 von mindestens 90%, noch stärker bevorzugt von mindestens 95%, noch stärker bevorzugt von mindestens 98% auf. Insbesondere weisen RNA1 und RNA2 jeweils die SEQ ID NO: 1 auf.

Noch stärker bevorzugt weisen RNA1 und RNA2 jeweils eine Sequenzhomologie zu SEQ ID NO: 1 von mindestens 90%, noch stärker bevorzugt von mindestens 95%, noch stärker bevorzugt von mindestens 98% auf. Insbesondere weisen RNA1 und RNA2 jeweils die SEQ ID NO: 1 auf.

Alternativ kann/können RNA1 und/oder RNA2 auch eine Sequenzhomologie zu SEQ ID NO: 2 mindestens 80%, bevorzugt von mindestens 90%, noch stärker bevorzugt von mindestens 95%, noch stärker bevorzugt von mindestens 98% aufweisen.

Bevorzugt weist RNA3 eine Sequenzhomologie zu SEQ ID NO: 2 von mindestens 80% auf:
5'-AAGAUGAACUUCAGGGUCAGCGUC-3' (SEQ ID NO: 2)

Stärker bevorzugt weist RNA3 eine Sequenzhomologie zu SEQ ID NO: 2 von mindestens 90%, noch stärker bevorzugt von mindestens 95%, noch stärker bevorzugt von mindestens 98% auf. Insbesondere weist RNA3 die SEQ ID NO: 2 auf.

Noch stärker bevorzugt weisen RNA3 und RNA4 jeweils eine Sequenzhomologie zu SEQ ID NO: 2 von mindestens 80% auf. Noch stärker bevorzugt weisen RNA3 und RNA4 jeweils eine Sequenzhomologie zu SEQ ID NO: 2 von mindestens 90%, noch stärker bevorzugt von mindestens 95%, noch stärker bevorzugt von mindestens 98% auf. Insbesondere weisen RNA3 und RNA4 jeweils die SEQ ID NO: 2 auf.

Alternativ kann/können RNA3 und/oder RNA4 auch eine Sequenzhomologie zu SEQ ID NO: 1 mindestens 80%, bevorzugt von mindestens 90%, noch stärker bevorzugt von mindestens 95%, noch stärker bevorzugt von mindestens 98% aufweisen.

Gemäß einer besonders bevorzugten Ausführungsform weisen RNA1 und RNA2 oder RNA3 und RNA4 jeweils eine Sequenzhomologie zu SEQ ID NO: 1 von mindestens 80% und RNA3 und RNA4 oder RNA1 und RNA2 jeweils eine Sequenzhomologie zu SEQ ID NO: 2 von mindestens 80% auf.

Gemäß einer ganz besonders bevorzugten Ausführungsform weisen RNA1 und RNA2 jeweils eine Sequenzhomologie zu SEQ ID NO: 1 von mindestens 80% und RNA3 und RNA4 jeweils eine Sequenzhomologie zu SEQ ID NO: 2 von mindestens 80% auf.

Alternativ können RNA1 und RNA2 jeweils eine Sequenzhomologie zu SEQ ID NO: 2 von mindestens 80% und können RNA3 und RNA4 jeweils eine Sequenzhomologie zu SEQ ID NO: 1 von mindestens 80% aufweisen.

Ganz besonders bevorzugt weisen RNA1 und RNA2 jeweils die Sequenz SEQ ID NO: 1 und RNA3 und RNA4 jeweils die Sequenz SEQ ID NO: 2 auf.

Alternativ können RNA1 und RNA2 jeweils die Sequenz SEQ ID NO: 2 und RNA3 und RNA4 jeweils die Sequenz SEQ ID NO: 1 aufweisen.

Offenbart wird das Oligonukleotid-Konjugat K oder pharmazeutische Zusammensetzung zur Verwendung in einem Verfahren zum Behandeln oder Vorbeugen einer Neoplasie, einer Infektion und/oder einer unkontrollierten Immunantwort.

Diesbezüglich gelten die obigen Definitionen und Ausführungen entsprechend.

In anderen Worten betrifft die vorliegende Offenbarung auch ein Verfahren zum Behandeln oder Vorbeugen einer Neoplasie, einer Infektion und/oder einer unkontrollierten Immunantwort in einem Patienten, wobei das Verfahren die Verabreichung einer geeigneten Dosis des Oligonukleotid-Konjugats K oder einer pharmazeutische Zusammensetzung an den Patient umfasst.

Wie hierin verwendet ist unter einem Patienten im weitesten Sinne jedes Individuum zu verstehen, das durch ein erfindungsgemäßes Oligonukleotid-Konjugat K behandelt werden soll oder einer vorbeugenden Maßnahme basierend auf einem erfindungsgemäßen Oligonukleotid-Konjugat K unterzogen werden soll. Daher weist der Patient bevorzugt eine Neoplasie, eine Infektion und/oder eine unkontrollierte Immunantwort oder ein Risiko mindestens eines der vorgenannten zu entwickeln auf. Der Patient kann ein beliebiges Tier, einschließlich eines Menschen sein. Bevorzugt ist der Patient ein Säugetier (z.B. ein Mensch, eine Maus, eine Ratte, ein Rind, ein Schwein, ein Hund, eine Katze oder ein Pferd usw.). Am stärksten bevorzugt ist der Patient ein Mensch.

Bei einer Neoplasie, einer Infektion und/oder einer unkontrollierten Immunantwort können optional klinische Symptome erkennbar sein, treten aber nicht zwingend auf, sondern können auch latent (daher auch unentdeckt) auftreten. Eine Neoplasie, eine Infektion und/oder eine unkontrollierten Immunantwort kann akut und/oder chronisch (daher beispielsweise mindestens einen Monat lang, mindestens sechs Monate lang oder mindestens ein Jahr lang) auftreten.

Wie hierin verwendet ist eine Neoplasie im weitesten Sinne als ein beliebiges Gewebe zu verstehen, das zu fehlkontrolliertem zellulären Wachstum neigt. In vielen Fällen neigt eine Neoplasie zu der Bildung einer Tumormasse, die optional von Blutgefäßen innerviert sein kann. Es können auch, müssen aber nicht, ein oder mehr Metastasen auftreten.

Eine Neoplasie im Sinne der vorliegenden Erfindung kann jede beliebige Neoplasie sein, wie sie nach der Internationalen statistischen Klassifikation der Krankheiten und verwandter Gesundheitsprobleme Version 10 (engl. International Statistical Classification of Diseases and Related Health Problems 10^{th} Revision (ICD-10) als eine der ICD-10-Klassen C00-D48 definiert wird.

Beispielhaft kann eine Neoplasie in diesem Zusammenhang eine Neoplasie ausgewählt aus einer malignen Neoplasie (Tumor) (ICD-10-Klassen C00-C97), einer *In situ*-Neoplasie (ICD-10-Klassen D00-D09), einer gutartigen Neoplasie (ICD-10-Klassen D10-D36) oder einer Neoplasie unsicheren oder unbekannten Verhaltens (ICD-10-Klassen D37-D48) sein.

Gemäß einer bevorzugten Ausführungsform ist die Neoplasie die Entstehung oder das Vorhandenseins eines Tumors.

Bevorzugt ist die Neoplasie eine Krebserkrankung. Krebs kann einen oder mehrere solide Tumoren umfassen und/oder eine hämatologische Erkrankung sein. Bevorzugt spricht die Neoplasie (grundsätzlich) auf immuntherapeutische Ansätze an wie beispielsweise therapeutische, gegen die Neoplasie gerichtete Antikörper oder Analogons davon.

Neoplasien können auf unterschiedliche Arten eingeteilt und klassifiziert werden. So können sie etwa nach der Lokalisation ihrer Hauptmasse (insbesondere etwa eines soliden Tumors) oder aber nach der ursprünglichen geweblichen Herkunft der Neoplasie (daher von dem Gewebe, von dem sich die Neoplasie ableitet) charakterisiert und klassifiziert werden.

Ein Aspekt der vorliegenden Erfindung betrifft das Oligonukleotid-Konjugat K gemäß der vorliegenden Erfindung zur Verwendung in einem Verfahren zum Behandeln oder Vorbeugen eines Tumors.

Die Neoplasie ist bevorzugt eine maligne Neoplasie (ICD-10-Klassen C00-C97). Eine Neoplasie kann etwa ausgewählt sein aus der Gruppe bestehend aus bösartigen Neubildungen an genau bezeichneten Lokalisationen (ICD-10-Klassen C00-C75) (z.B. an Lippe, Mundhöhle oder Pharynx (ICD-10-Klassen C00-C14), an einem Verdauungsorgan (ICD-10-Klassen C15-C26), an einem Atmungsorgan oder sonstigen intrathorakalen Organen (ICD-10-Klassen C30-C39), an Knochen oder Gelenkknorpel (ICD-10-Klassen C40-C41), an der Haut (ICD-10-Klassen C43-C44), an mesothelialem Gewebe oder Weichteilgewebe (ICD-10-Klassen C45-C49), an der Brustdrüse (ICD-10-Klasse C50), an weibliche Genitalorganen (ICD-10-Klassen C51-C58), an männlichen Genitalorganen (ICD-10-Klassen C60-C63), an einem Harnorgan (ICD-10-Klassen C64-C68), an Auge, Gehirn oder einem sonstigen Teil des Zentralnervensystems (ICD-10-Klassen C69-C72) oder an Schilddrüse oder sonstigen endokrinen Drüsen (ICD-10-Klassen C73-C75)), bösartige Neubildungen ungenau bezeichneter, sekundärer und nicht näher bezeichneter Lokalisationen (ICD-10-Klassen C76-C80), bösartige Neubildungen des lymphatischen, blutbildenden und verwandten Gewebes (ICD-10-Klassen C81-C96) und bösartige Neubildungen als Primärtumoren an mehreren Lokalisationen (ICD-10-Klasse C97).

Die Neoplasie kann ausgewählt sein aus der Gruppe bestehend aus einem Karzinom (d.h. Krebsarten, die sich ableiten von epithelialen Zellen wie beispielsweise ein Adenokarzinom, ein Plattenepithelkarzinom, ein anaplastisches Karzinom, ein großzelliges oder kleinzelliges (Lungen)karzinom), einem Sarkom (d.h. Krebsarten, die sich ableiten von Bindegewebe wie beispielsweise einem Askintumor, Sarkoma botryoides, einem Chondrosarkom, einem Ewing-Sarkom, einem malignem Hemangioendotheliom, malignem Schwannom, einem Osteosarkom, einem Weichteilsarkom), einem Lymphom und Leukämie (d.h. Krebsarten, die sich von hämatopoietischen (blutbildenden) Zellen ableiten wie etwa B-Zell-Neoplasien, T-Zell-Neoplasien, natürliche Killerzell-Neoplasien, Hodgkin-Lymphom, Nicht-Hodgkin-Lymphom, immundefizienz-assoziierte lymphoproliferative Störungen, lymphozytische Leukämie, myelogene Leukämie), einem Keinzelltumor (d.h. Krebsarten, die sich ableiten von pluripotenten Zellen der Sexualorgane wie etwa ein Germinom (einschließlich Dysgerminom und Seminom), einem Blastom (d.h. Krebsarten, die sich ableisten von unreifen Vorläuferzellen oder embryonischem Gewebe wie etwa ein Hepatoblastom, ein Medulloblastom, ein Nephroblastom, ein Neuroblastom, ein Pancreatoblastom, ein pleuropulmonares Blastom, ein Retinoblastom, ein Glioblastom), einem Melanom und Vorstufen davon (d.h. Krebsarten, die sich ableiten von Melanozyten wie beispielsweise Lentigo maligna, Superficial-Spreading-Melanom, acrales lentigines Melanom, Schleimhautmelanom, nodulares Melanom, polypoides Melanom, desmoplastisches Melanom, amelanotides Melanom, Weichteilmelanom), nicht-melanomen Hautkrebs (d.h. nicht-melanomen Krebsarten, die sich von der haut ableiten wie beispielsweise Basalzellencarcinom, Plattenepithelkarzinom, Dermatofibrosarkom protuberans, Merkel-Zell-Karziniom, Kaposi-Sarkom, Keratoacanthom, Spindelzelltumor) und einem Gliom (d.h. Krebsarten, die sich ableiten von Hirn- oder Rückenmarkzellen, wie beispielsweise Ependymom, Astrocytom, Oligodendrogliom, Hirnstammgliom, Sehnervgliom, gemischtes Gliom).

Eine Infektion kann im Sinne der vorliegenden Offenbarung jede beliebige Infektion sein. Infektion sollte im weitesten Sinne verstanden werden als ein pathologischer Zustand, der durch die Invasion eines Pathogens oder mehrerer Pathogene hervorgerufen wird. Das Pathogen ist hierbei in der Regel ein biologisches Pathogen. Hierbei kann es sich etwa um eine Infektion durch Viren, Bakterien, Pilze, Viroide, Prionen, eukaryotische Mikroorganismen, Nematoden, Rundwürmer, Plattwürmer, Bandwürmer und/oder Arthropoden (z.B., Insekten, Milben, Zecken, Läuse, Flöhe) handeln.

Ein Aspekt der vorliegenden Erfindung betrifft das Oligonukleotid-Konjugat K zur Verwendung in einem Verfahren zum Behandeln oder Vorbeugen einer viralen Infektion.

Eine virale Infektion kann akut oder chronisch sein. Nichtbeschränkte Beispiele für virale pathogene sind beispielsweise Pockenvirus, Ebola-Virus, Lassa-Virus, Marburg-Virus, Adenovirus, Herpes-Simplex, type 1, Herpes-Simplex, type 2, Varicellazostervirus, Epstein-Barr-Virus, Humanes Cytomegalovirus, Humanes Herpesvirus, type 8, Humanes Papillomavirus, BK-virus, JC-Virus, Smallpox-Virus, Hepatitis B-Virus, Hepatitis C-Virus, Hepatitis D-Virus, Hepatitis E-Virus, Humanes Bocavirus, Parvovirus B19, Humanes Astrovirus, Norwalk-Virus, Coxsackievirus, Hepatitis A-Virus, Poliovirus, Rhinovirus, Schweres Akutes Atemwegssyndrom-Virus (SARS), Gelbfiebervirus, Denguevirus, West-Nil-Virus, Rubella-Virus, HIV-1, HIV-2, Grippevirus, Guanarito-Virus, Junin-Virus, Machupo-Virus, Sabiä-Virus, Crimean-Congo-Hemorrhagisches-Fieber-Virus, Masernvirus, Mumpsvirus, Parainfluenza-Virus, Rotavirus, Orbivirus, oltivirus und Banna-Virus.

Eine unkontrollierte Immunantwort kann eine Überreaktion oder eine Unterreaktion des Immunsystems sein.

Der Fachmann wird erkennen, dass die Dosis, die dem Patienten verabreicht wird, abhängig von der Art des zu behandelnden Leiden, dem Zustand des Patienten wie auch dem Gewicht des Patienten, dem Alter des Patienten und der Art und Schwere der Erkrankung ist.

Das beschriebene Oligonukleotid-Konjugat K wird bevorzugt in Form einer pharmazeutischen Zusammensetzung an den Pateinten verabreicht werden. Die therapeutische Wirksamkeit und Toxizität kann nach Standardprotokollen bestimmt werden.

Die pharmazeutische Zusammensetzung kann systemisch verabreicht werden, wie beispielsweise intraperitoneal, intramuskulär oder intravenös oder lokal, wie beispielsweise intranasal, subkutan, intradermal oder intrathekal. In einer bevorzugten Ausführungsform wird die pharmazeutische Zusammensetzung intradermal verabreicht. Dazu kann beispielsweise ein Tätowier-Verfahren wie in WO 2014/049079 (vgl. beispielsweise Seiten 13-15 hieraus) beschrieben verwendet werden.

Das erfindungsgemäße Oligonukleotid-Konjugat K zur Verwendung in einem Verfahren zum Behandeln oder Vorbeugen eines Tumors und/oder einer viralen Infektion kann stabilisiert werden, indem es Teil einer pharmazeutischen Zusammensetzung ist.

Daher betrifft die vorliegende Erfindung auch eine pharmazeutische Zusammensetzung umfassend ein Oligonukleotid-Konjugat K gemäß der vorliegenden Erfindung und einen pharmazeutisch verträglichen Träger zur Verwendung in einem Verfahren zum Behandeln oder Vorbeugen eines Tumors und/oder einer viralen Infektion.

Ein pharmazeutisch verträglicher Träger kann ein beliebiger Stoff sein, der in der eingesetzten Menge keine toxische Wirkung auf den Patienten hat und daher für diesen verträglich ist. Beispielsweise kann ein pharmazeutisch verträglicher Träger ein Lösungsmittel wie z.B. Wasser, Dimethylsulphoxid (DMSO), Ethanol, Pflanzenöl, Paraffinöl oder eine Kombination aus zwei oder mehr daraus sein.

Eine pharmazeutische Zusammensetzung kann optional auch ein oder mehrere Detergenz(ien) (z.B., Natriumlaurylsulfat (SLS)/Natriumdodecylsulfat (SDS)), ein oder mehrere Farbstoff(e) (z.B., TiO₂, Nahrungsfarbstoff), ein oder mehrere Vitamin(e), ein oder mehrere Salze(e) (z.B. Natrium-, Kalium-, Magnesium-, Kalzium- und/oder Zinksalze), ein oder mehrere Feuchthaltemittel (z.B. Sorbitol, Glycerin, Mannitol, Propylenglycol, Polydextrose), ein oder mehrere Enzym(e), ein oder mehrere Konservierungsmittel (z.B. Benzoesäure, Methylparaben), ein oder mehrere Verdickungsmittel (z.B. Carboxymethylzellulose (CMC), Polyethylenglycol (PEG), Sorbitol), ein oder mehrere Lösungsvermittler, ein oder mehrere Emulsionsmittel, ein oder mehrere Füllstoff(e), ein oder mehrere Glanzvermittler, ein oder mehrere Trennmittel, ein oder mehrere Antioxidantien, ein oder mehrere Pflanzenextrakte, ein oder mehrere Stabilisierungsmittel, ein oder mehrere Polymere (z.B. Hydroxypropylmethacrylamid (HPMA), Polyethylenimine (PEI), Carboxymethylzellulose (CMC), Polyethylenglycol (PEG)), ein oder mehrere Aufnahmevermittler (z.B. Polyethylenimin (PEI), Dimethylsulphoxide (DMSO), ein zellpenetrierendes Peptid (CPP), eine Proteintransduktionsdomäne (PTD), ein antimicrobielles Peptid usw.) ein oder mehrere Antikörper, ein oder mehrere Geschmacksstoffe (z.B. Süßstoffe), ein oder mehrere fluoreszente Stoffe und/oder ein oder mehrere Duftstoffe umfassen. Erkennbar können einige Stoffe auch mehrere Funktionen erfüllen.

Das offenbarte Oligonukleotid-Konjugat K kann optional auch in eine makromolekulare Struktur eingebettet werden (z.B. ein Liposom, ein Polymerosom, eine Mizellstruktur, ein (unstrukturiertes) ausgehärtetes Polymer (z.B. Eudragit) usw.). Dies wird in der Regel nicht-kovalent geschehen. Alternativ oder zusätzlich kann das erfindungsgemäße Oligonukleotid-Konjugat K auch kovalent oder nicht-kovalent an ein Polypeptid, ein Polymer, ein Harzkügelchen usw. gebunden sein. Derartige Formulierungen können die Halbwertszeit *in vivo* sowie die Haltbarkeit erheblich verlängern. Zudem kann hierdurch die Aufnahmerate in den Körper und in Zellen erheblich verbessert werden.

Sowohl *in vivo* wie auch *in vitro* kann die Aufnahmerate in den Körper und in Zellen durch Formulierung mit und/oder kovalente Anknüpfung an Aufnahmevermittler (z.B. Polyethylenimin (PEI), Dimethylsulphoxide (DMSO), ein zellpenetrierendes Peptid (CPP), eine Proteintransduktionsdomäne (PTD), ein antimicrobielles Peptid usw.) verbessert werden. Möglichst wird die Aufnahme in das Zytosol der Zielzellen erreicht. Aufnahmevermittler sollten die Aktivität des erfindunsggemäßen Oligonukleotid-Konjugats K (z.B. die RIG-I-Aktivierung) möglichst nicht oder nur unwesentlich stören oder als Vorläuferverbindung (engl. prodrug) die Freisetzung des aktiven RNA-Liganden ermöglichen, etwa innerhalb des Zytosols. Als nichtkovalente oder kovalente Aufnahmevermittler können ebenfalls dienen:
- Liposomen,
- Mizellen,
- polymere kationische Lipide wie Polyethylenimine,
- Monosaccharide, Disaccharide und andere Kohlenhydrate wie beispielsweise Glukose, Mannose, Galaktose, Dextrine oder Cyclodextrine usw.,
- nichtionische oberflächenaktive Stoffe wie Polyethylen oder Polyethylenglykol,
- hydrophile Polymere wie Polyacrylate.
Darüber hinaus kann die Formulierung der RNA-Liganden Hilfsstoffe zur Stabilisierung der RNA-Liganden enthalten, wie Chelatbildner, Salze oder RNasen-inhibierende Substanzen.

Ein Oligonukleotid-Konjugat K kann erfindungsgemäß grundsätzlich auch *in vitro* zur Induktion vermehrter Sezernierung von Interferon-alpha verwendet werden.

Sowohl im medizinischen Kontext sowie auch im nicht-medizinischen Kontext, *in vivo* und *in vitro,* kann das Oligonukleotid-Konjugat K als einziger pharmazeutisch aktiver Wirkstoff oder in Kombination mit einem oder mehreren anderen pharmazeutisch aktiven Wirkstoff(en) eingesetzt werden. Dies können etwa antiproliferative/antineoplastischen und antivirale Wirkstoffe sein. Beispielsweise könne derartige Wirkstoffe ausgewählt sein aus der Liste bestehend aus Purinantagonisten und Pyrimidinbasenanagonsiten, polyklonalen oder monoklonalen Antikörpern (z.B. Rituximab, Trastuzumab, Cetuximab, Bevacizumab, Basiliximab, Daclizumab), Antimetaboliten (z.B. 5-Fluorouracil, Azathioprin, 6-Mercaptopurin, Mercaptopurin, Pyrimidine, Thioguanin, Fludarabin, Floxuridin, Cytosine Arabinosid (Cytarabin), Pemetrexed, Raltitrexed, Pralatrexat, Methotrexat), Antiestrogene, Hormone und Hormonantagonisten, Alkylantien (z.B. Mechlorethamin, Cyclophosphamid, Chlorambucil, Ifosfamid), Platine (z.B. Cisplatin, Carboplatin, Oxaliplatin), Pflanzenalkaloide und Terpenoide (z.B. Vincaalkaloids (Vincristin, Vinblastin, Vinorelbin, Vindesin), Taxane (z.B. Paclitaxel), Cytoxan), Topoisomerase-Inhibitoren (z.B. Camptothecine: Irinotecan, Topotecan, Etoposid, Etoposid Phosphat, Teniposid), Melphalan, Antineoplastica (z.B. Doxorubicin (adriamycin), Doxorubicin Lipo, Epirubicin, Bleomycin)), Actinomycin D, Aminoglutethimid, Amsacrin, Anastrozol, Anthracycline, Aromatase-Inhibitoren, Asparaginas, Bexaroten, Buserelin, Busulfan, Camptothecinderivate, Capecitabin, Carmustin, Cladribin, Cytarabin, Cytosinarabinosid, alkyliende Zytostatica, Dacarbazin, Daunorubicin, Docetaxel, Epirubicin, Estramustin, Etoposid, Exemestan, Fludarabin, Fluorouracil, Folsäureantagonisten, Formestan, Gemcitabin, Glucocorticoide, Goserelin, Hycamtin, Hydroxyurea, Idarubicin, Irinotecan, Letrozol, Leuprorelin, Lomustin, Mercaptopurin, Miltefosin, Mitomycine, Mitoseinhibitoren, Mitoxantron, Nimustin, Procarbazin, Tamoxifen, Temozolomid, Teniposid, Testolacton, Thiotepa, Topoisomerase Inhibitors, Treosulfan, Tretinoin, Triptorelin, Trofosfamid, zytostatisch aktive Antibiotica, Everolimus, Pimecrolimus, Tacrolimus, Azithromycin, Spiramycin, Sirolimus (rapamycin), Roxithromycin, Ascomycin, Bafilomycin, Erythromycin, Midecamycin, Josamycin, Concancamycin, Clarithromycin, Troleandomycin, Folimycin, Tobramycin, Mutamycin, Dactinomycin, Dactinomycin, Rebeccamycin, A Statin (z.B. Cerivastatin, Simvastatin, Lovastatin, Somatostatin, Fluvastatin, Nystatin, Rosuvastatin, Atorvastatin, Pravastatin, Pitavastatin, Pentostatin), 4-Hydroxyoxycyclophosphamid, Bendamustin, Thymosin α-1, Aclarubicin, Fludarabine-5'-dihydrogen Phosphat, Hydroxycarbamid, Aldesleukin, Pegaspargas, Cepharanthin, Epothilon A und B, Azathioprin, Mycophenolat, Mofetil, C-myc-Antisens, B-myc-Antisens, Camptothecin, Melanozyten Stimuliendes Hormon (a-MSH), aktiviertes Protein C, IL-1β-Inhibitor, Fumarsäure und deren Ester, Dermicidin, Calcipotriol, Taclacitol, Lapachol, β-Lapachon, Podophyllotoxin, Betulin, Podophyllinsäure, 2-Ethylhydrazid, Sagramostim, (rhuGM-CSF), Peginterferon α-2b, Lenograstim (r-HuG-CSF), Filgrastim, Macrogol, Cephalomannin, Selectin (Zytokinantagonist), CETP-Inhibitor, Cadherins, Cytokinin Inhibitors, COX-Inhibitor (z.B. COX-2- oder COX-3- Inhibitor), Angiopeptin, Ciprofloxacin, Fluroblastin, BFGF-Antagonisten, Probucol, Prostaglandine, 1,11-Dimethoxyeanthin-6-on, 1-Hydroxy-11-methoxycanthin-6-on, Scopoletin, Colchicin, NO Donors, Pentaerythrityl Tetranitrat, Sydnonimine, S-Nitrosoderivate, Staurosporin, β-Estradiol, α-Estradiol, Estriol, Estron, Ethinyl Estradiol, Fosfestrol, Medroxyprogesteron, Estradiol Cypionates, Estradiot Benzoates, Tranilast, Kamebakaurin, Verapamil, Ciclosporin A, Paclitaxel und dessen Derivate wie etwa 6-α-Hydroxypaclitaxel, Baccatin, Taxoter, Mofebutazon, Acemetacin, Diclofenac, Lonazolac, Dapson, O-Carbamoylphenoxyazetat, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-sitosterol, Ademetionin, Myrtecain, Polidocanol, Nonivamid, Levomenthol, Benzocain, Aescin, Elipticin, Calbiochem D-24851, Colcemid, Cytochalasin A-E, Indanocin, Nocodazol, Bacitracin, Vitronectin-Rezeptor-Antagonisten, Azelastin, freie Nukleinsäuren, in Virustransmitter inkorporierte Nukleinsäuren, andere DNA- oder RNA-Fragmente, Plasminogen Activator Inhibitor-1, Plasminogen Aktivierender Inhibitor-2, Antisense Oligonucleotid, VEGF-Inhibitoren, IGF-1, Wirkstoffe aus der Gruppe der Antibiotika wie etwa Cefadroxil, Cefazolin, Cefaclor, Cefoxitin, Gentamicin, Penicillins, Dicloxacillin, Oxacillin, Sulfonamide, Metronidazol, Antithrombotica, Argatroban, Aspirin, Abciximab, synthetisches Antithrombin, Bivalirudin, Coumadin, Enoxaparin, GpIIb/IIIa Platelet Membrane Receptor, Antikörper gegen Factor Xa Inhibitor, Heparin, Hirudin, R-hirudin, PPACK, Protamin, Prourokinas, Streptokinas, Warfarin, Urokinas, Vasodilators, Dipyramidol, Trapidil, Nitroprussides, PDGF-Antagonisten, Triazolopyrimidin, Seramin, ACE-Inhibitoren, Captopril, Cilazapril, Lisinopril, Enalapril, Losartan, Thioprotease-Inhibitoren, Prostacyclin, Vapiprost, Interferon-a, -β und -γ, Histaminantagonisten, Serotoninblocker, Apoptoseinhibitoren, Apoptoseregulatoren, NF-kB oder Bcl-xL-Antisenseoligonucleotide, Halofuginon, Nifedipin, Tocopherol, Molsidomin, Teepolyphenole, Epicatechin Gallat, Epigallocatechin Gallat, Boswellinsäure und deren Derivate, Leflunomid, Anakinra, Etanercept, Sulfasalazin, Tetracyclin, Triamcinolon, Procainimid, Retinoinsäure, Quinidin, Disopyramid, Flecainid, Propafenon, Sotalol, Amiodaron, natürliche und synthetische Steroide wie etwa Bryophyllin A, Inotodiol, Maquiroside A, Mansonin, Streblosid, Hydrocortison, Betamethason, Dexamethason, Fenoprofen, Ibuprofen, Indomethacin, Naproxen, Phenylbutazon, Acyclovir, Ganciclovir, Zidovudin, Antimycotics, Clotrimazol, Flucytosin, Griseofulvin, Ketoconazol, Miconazol, Terbinafin, Chloroquin, Mefloquin, Quinin, natürliche Terpenoide, Hippocaesculin, Barringtogenol-C21-angelate 14-dehydroagrostistachin, Agroskerin, Agrostistachin, 17-hydroxyagrostistachin, Ovatodiolids, 4,7-Oxycycloanisomelinsäure, Baccharinoide B1, B2, B3 And B7, Tubeimosid, Bruceanol A, B And C, Bruceantinoside C, Yadanziosides N und P, Isodeoxyelephantopin, Tomenphantopin A und B, Coronarin A, B, C And D, Hyptatic Acid A, Zeorin, Iso-iridogermanal, Maytenfoliol, Effusantin A, Excisanin A And B, Longikaurin B, Sculponeatin C, Kamebaunin, Leukamenin A And B, 13,18-Dehydro-6-alpha-senecioyloxychaparrin, Taxamairin A And B, Regenilol, Triptolid, Cymarin, Apocymarin, Aristolochiasäure, Anopterin, Hydroxyanopterin, Anemonin, Protoanemonin, Berberin, Cheliburinchlorid, Cicutoxin, Sinococulin, Combrestatin A And B, Cudraisoflavone A, Curcumin, Dihydronitidin, Nitidinchlorid, 12-Beta-hydroxypregnadiene-3,20-dione Bilobol, Ginkgol, Ginkgolinsäure, Helenalin, Indicin, Indicine-N-oxid, Lasiocarpin, Inotodiol, Glycoside 1a, Justicidin A And B, Larreatin, Malloterin, Mallotochromanol, Isobutyrylmallotochromanol, Marchantin A, Maytansin, Lycoridicin, Margetin, Pancratistatin, Liriodenin, Bisparthenolidin, Oxoushinsunin, Aristolactam-All, Periplocoside A, Ghalakinosid, Deoxypsorospermin, Psychorubin, Ricin A, Sanguinarin, Manwuweizensäure, Methylsorbifolin, Chromones von Spathelia, Stizophyllin, Akagerin, Dihydrousambaraensin, Hydroxyusambarin, Strychnopentamin, Strychnophyllin, Usambarin, Usambarensin, Daphnoretin, Lariciresinol, Methoxylariciresinol, Syringaresinol, Umbelliferon, Afromoson, Acetylvismione B, Desacetylvismione A, Vismione A und B), antiangiogenische Wirkstoffe (z.B. Carboxyamidotriazol, TNP-470, CM101, Suramin, SU5416, Thrombospondin, VEGFR-Antagonisten, angiostatische Steroide + Heparin), Matrixmetalloproteinase-Inhibitoren, 2-Methoxyestradiol, Tecogalan, Tetrathiomolybdat, Thalidomid, Thrombospondin, Angiopoietin 2, Angiostatin (z.B. TSP-1 And TSP-2 Angiostatin), Endostatin, Vasostatin, Canstatin, Calreticulin, Platelet Factor-4, TIMP And CDAI, Meth-1 und Meth-2, Kinaseinhibitoren (z.B. Imatinib, Imatinib Mesylat, Gefitinib, Erlotinib, Pazopanib, Apatinib), Proteasome-Inhibitoren (z.B. Bortezomib), PARP-Inhibitoren (z.B. Iniparib, Olaparib), und Kombinationen von zwei oder mehr daraus.

Auch kann Strahlentherapie eingesetzt und somit auch Iod-131, Lutetium-177, Strontium-89, Samarium (¹⁵³Sm), Lexidronam und/oder Yttrium-90) verwendet werden.

Daher betrifft ein weiterer Aspekt der vorliegenden Erfindung die Verwendung eines Oligonukleotid-Konjugats K gemäß der vorliegenden Erfindung zur Induktion vermehrter Sezernierung von Interferon-alpha *in vitro.*

Bezüglich dieses Aspekts gelten die obigen Definitionen und Ausführungen entsprechend.

Hierbei kann das beschriebene Oligonukleotid-Konjugat K beispielsweise Zellen einer Zellkultur zugegeben werden. Diese können dann nach einer geeigneten Inkubationszeit vermehrt proinflamatorische Zytokine sezernieren und/oder werden apoptotisch.

Als bevorzugtes Beispiel eines proinflammatorischen Zytokins sei Interferon alpha (INFα) genannt, wie es auch experimentell gemessen wurde (siehe Beispielteil).

Das beschriebene Oligonukleotid-Konjugat K kann auf beliebige Weise erhalten werden. Gegenüber anderen Oligonukleotid-Konjugaten, die einzelne ähnliche strukturelle Merkmale aufweisen, ergeben sich jedoch auch besondere synthetische Vorteile.

Gelehrt wird auch ein Verfahren zum Herstellen eines Oligonukleotid-Konjugats K gemäß der vorliegenden Offenbarung, umfassend die folgenden Schritte:
(ia) parallele Festphasensynthese von geschützten Vorläufern von RNA1 und RNA2 auf einem gemeinsamen polymeren Träger, wobei die geschützten Vorläufer von RNA1 und RNA2 jeweils mit einem gleichartigen Terminus an den Träger gebunden sind;
(iia) Entschützen der vom polymeren Träger abgewandten Termini der geschützten Vorläufer aus Schritt (ia);
(iiia) Verknüpfen eines aktivierten Vorläufers des bivalenten Linkers B mit den freien Termini der terminal entschützten Vorläufer von RNA1 und RNA2 aus Schritt (iia) oder aktivierter Vorläufer aus den terminal entschützten Vorläufern von RNA1 und RNA2 aus Schritt (iia) mit einem bivalenten Linker B zu einem Konjugat RNA1-B-RNA2;
(iva) Abspalten und Entschützen der Konjugate RNA1-B-RNA2 aus Schritt (iiia);
(va) Aufreinigen der Konjugate RNA1-B-RNA2 aus Schritt (iva); und
(via) Zugeben geeigneter Anteile an RNA3 und RNA4 zu dem Konjugat aus Schritt (va) unter Bedingungen, die ein Zusammenlagern der komplementären Stränge erlauben.

Bezüglich eines solchen Verfahrens gelten die obigen Definitionen und Ausführungen entsprechend.

Der Fachmann kann Schritte (ia) und (iia) mittels Standardverfahren zur Herstellung von Oligonukleotiden mittels Festphasensynthese durchführen. Das Verknüpfen nach Schritt (iiia) hängt vom jeweilig gewählten bivalenten Linker B ab. Auch hierbei wird der Fachmann auf Routineverfahren zurückgreifen. Meist wird hierbei entweder der bivalente Linker B und/oder die freien Termini der terminal entschützten Vorläufer von RNA1 und RNA2 chemisch mittels eines Kopplungsagenz aktiviert und dann gekoppelt. Abspalten, Entschützen und Aufreinigen nach Schritten (iva) und (va) erfolgt nach Routineverfahren aus der Festphasensynthese von Oligonukleotiden. Das Zugeben geeigneter Anteile an RNA3 und RNA4 (Schritt (via)) erfolgt bevorzugt in weitgehend neutraler wässriger Umgebung (pH 6-7,5) bei Temperaturen zwischen 4°C und 75°C, so dass sich die komplementären Stränge gut an einander anlagern können.

Ferner offenbart ist ein alternatives Verfahren zum Herstellen eines Oligonukleotid-Konjugats K gemäß der vorliegenden Offenbarung, umfassend die folgenden Schritte:
(ib) Bereitstellen eines an eine Festphase gebundenen bivalenten Linkers B;
(iib) parallele Festphasensynthese von geschützten Vorläufern von RNA1 und RNA2 auf den an eine Festphase gebundenen bivalenten Linker B aus Schritt (ib), wobei die geschützten Vorläufer von RNA1 und RNA2 jeweils mit einem gleichartigen Terminus kovalent an den bivalenten Linker B gebunden sind;
(iiib) Abspalten und Entschützen der aus Schritt (iib) erhaltenen Konjugate RNA1-B-RNA2;
(ivb) Aufreinigen der Konjugate RNA1-B-RNA2 aus Schritt (iiib); und
(vb) Zugeben geeigneter Anteile an RNA3 und RNA4 zu dem Konjugat aus Schritt (ivb) unter Bedingungen, die ein Zusammenlagern der komplementären Stränge erlauben.

Bezüglich eines solchen Verfahrens gelten die obigen Definitionen und Ausführungen entsprechend.

Das Bereitstellen des an eine Festphase gebundenen bivalenten Linkers B (Schritt (ib)) kann auf beliebige Weise erfolgen. Der Fachmann wird erkennen, dass das genaue chemische Vorgehen vom jeweilig verwendeten bivalenten Linker B abhängt. Dem Fachmann sind zur Kopplung zahlreiche Standardverfahren bekannt. Schritte (iib)-(ivb) können mittels Standardvorgehen bei Festphasensynthese von Oligonukleotiden erfolgen. Das Zugeben geeigneter Anteile an RNA3 und RNA4 (Schritt (via)) erfolgt bevorzugt in weitgehend neutraler wässriger Umgebung (pH 6-7,5) bei Temperaturen zwischen 4°C und 75°C, so dass sich die komplementären Stränge gut an einander anlagern können. Bevorzugt wird kurz erhitzt, um Einzelstränge voneinander zu trennen, und dann die Temperatur gesenkt, um die komplementären Stränge aneinander anzulagern.

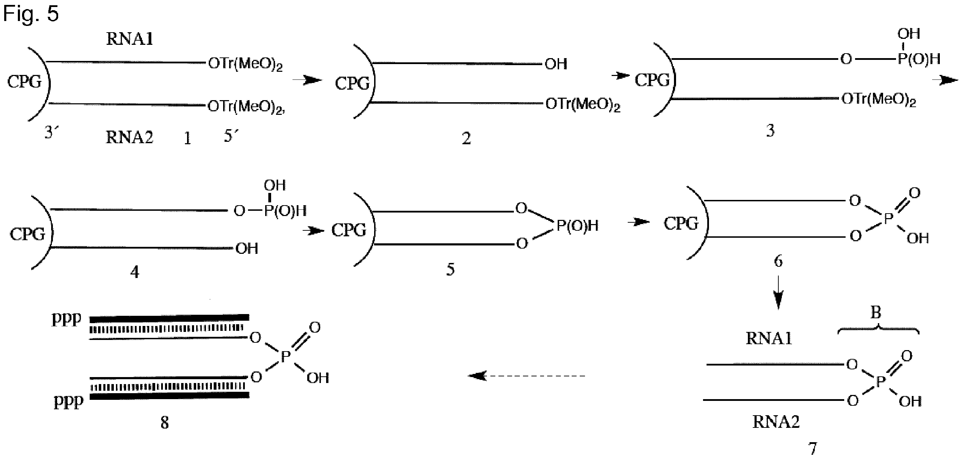

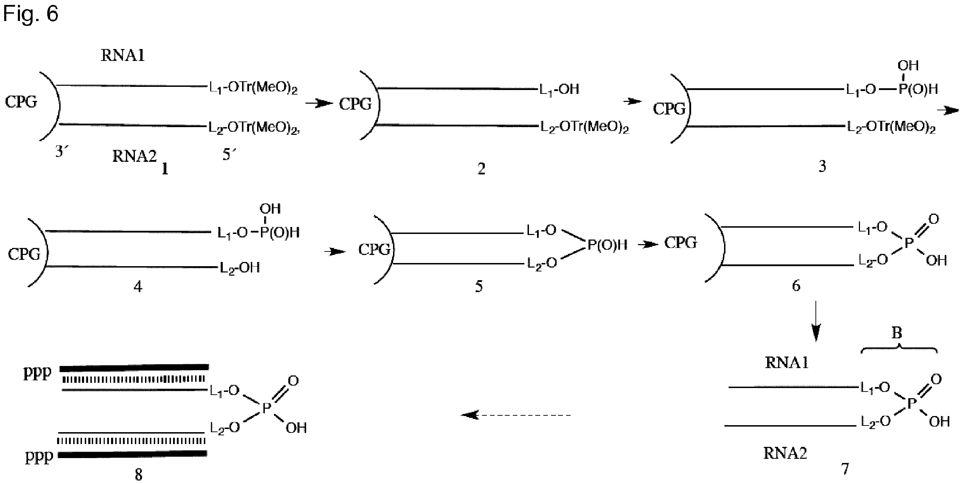

Eine weitere, alternative und bevorzugte Ausführungsform betrifft ein Verfahren zum Herstellen eines Oligonukleotid-Konjugats K wie in Figuren 5 und 6 gezeigt. Ein derartiges Verfahren umfasst die folgenden Schritte:
(ic) parallele Festphasensynthese von geschützten Vorläufern von RNA1 und RNA2 auf einem gemeinsamen polymeren Träger, wobei die geschützten Vorläufer von RNA1 und RNA2 jeweils mit einem gleichartigen Terminus an den Träger gebunden sind, wobei die Vorläufer von RNA1 und RNA2 optional an den vom polymeren Träger abgewandten Termini eine oder mehrere zusätzliche Linkerstrukturen L1 und/oder L2 tragen können;
(iic) partielles Entschützen von (etwa) 50% der vom polymeren Träger abgewandten Termini der geschützten Vorläufer aus Schritt (ic);
(iiic) monovalentes Verknüpfen eines aktivierten Vorläufers einer bivalenten Linkerstruktur B* mit einem freien Terminus eines terminal entschützten Vorläufers aus Schritt (iic) oder monovalentes Verknüpfen eines aktivierten Vorläufers eines terminal entschützten Vorläufers aus Schritt (iic) mit einer bivalenten Linkerstruktur B* zu festphasengebundenen Konjugaten RNA1-B* und/oder RNA2-B*;
(ivc) Entschützen des terminalen Endes der bislang nicht entschützten Vorläufer;
(vc) Konjugieren der zweiten, bislang nicht gebundenen Bindestelle einer bivalenten Linkerstruktur B* aus den festphasengebundenen Konjugaten RNA1-B* und/oder RNA2-B* mit dem entschützten terminalen Ende eines nach Schritt (ivc) entschützten Vorläufers zu einem Konjugat RNA1-B-RNA2;
(vic) Abspalten und Entschützen der Konjugate RNA1-B-RNA2 aus Schritt (vc);
(viic) Aufreinigen der Konjugate RNA1-B-RNA2 aus Schritt (vib); und
(viiic) Zugeben geeigneter Anteile an RNA3 und RNA4 zu dem Konjugat aus Schritt (viib) unter Bedingungen, die ein Zusammenlagern der komplementären Stränge erlauben.

Letztlich bildet die bivalenten Linkerstruktur B* optional zusammen mit L1 und/oder L2 den bivalenten linker B.

Bevorzugt sind hierbei RNA1 und RNA2 identisch, so dass das partielle Entschützen auch so erfolgen kann, dass Bedingungen gewählt werden, bei denen statistisch 50% der der festphasengebundenen Vorläufer aus Schritt (ic) auch ohne besondere Schutzgruppenstrategien verwendet werden können. RNA1 und RNA2 tragen dann bevorzugt die gleiche Schutzgruppe.

Alternativ kann mit orthogonalen Schutzgruppenstrategien gearbeitet werden (z.B. mit Fmoc-, Boc-, (MeO)₂-Trityl-Schutzgruppen usw.). In diesem Fall können dann auch gezielt RNA1 über den bivalenten Linker B mit RNA2 verknüpft werden. RNA1 und RNA2 tragen dann unterschiedliche Schutzgruppen, die selektiv unter unterschiedlichen Bedingungen abspaltbar sind und dadurch jeweils RNA1 oder RNA2 selektiv entschützen. Hierbei liegen RNA1 und RNA2 bevorzugt in einem äquimolaren Verhältnis vor.

Die entschützten funktionellen Gruppen, die miteinander verknüpft werden können, können auch als Donor und Akzeptor bezeichnet werden.

Beispielhaft kann die Verknüpfung der von der Festphase abgewandten terminalen Enden der Vorläufer von RNA1 und RNA2 miteinander (Schritte (iiic)-(vc)) über ein H-Phosphonat-Verfahren verknüpft werden wie es in Figuren 5 und 6 beispielhaft gezeigt wird. Wenn ein solches Verfahren eingesetzt wird, erlaubt dieses Verfahren optional auch weitere Derivatisierungen, welche aus der Reaktivität des P(III)-Linkers ableitbar sind. Die entsprechenden zahlreichen Möglichkeiten (z.B. Reaktionen mit Schwefelverbindungen, oxidative Amidierung usw.) sind dem Fachmann bekannt.

Als bivalenter Linkers B kann hierbei bevorzugt Phosphat oder ein Phosphitderivat verwendet werden.

Optionale zusätzliche Linkerstrukturen L1 und/oder L2 am vom polymeren Träger abgewandten Termini von RNA1 oder RNA2 können grundsätzlich beliebige bivalente Strukturen sein. Bevorzugt umfassen derartige Strukturen nicht mehr als 20 Kohlenstoffatome. Beispielhaft können derartige zusätzliche Linkerstrukturen L1 und/oder L2 Ethylenglycole (z.B. Hexaethylenglycol oder Triethylenglycol), Aminosäuren, C₁₋₁₀-Alkylspacer oder ähnliches sein.

Die Effizienz derartiger Verknüpfungsreaktionen zwischen zwei Reaktionspartnern, die an einem gemeinsamen polymeren Träger (Festphase) fixiert sind, ist üblicherweise vergleichsweise hoch. Zudem erfolgt die Festphasensynthese der Vorläufer von RNA1 und RNA2 üblicherweise in guten Ausbeuten und hoher Reinheit. Ebenso kann die Ausbeute und die Reinheit des Konjugats RNA1-B-RNA2 und letztlich auch die Ausbeute und die Reinheit des Oligonukleotid-Konjugats K der vorliegenden Erfindung gemäß einer solchen Synthesestrategie besonders hoch sein.

Wie bereits oben ausgeführt, kann der Fachmann für die praktische Ausführung der einzelnen Schritte auf allgemeines Fachwissen zurückgreifen.

Die nachfolgend gezeigten Beispiele und Figuren sollen der Verdeutlichung der Erfindung dienen, aber nicht der Schutzbereich der Ansprüche beschränken.

### Kurze Beschreibung der Figuren

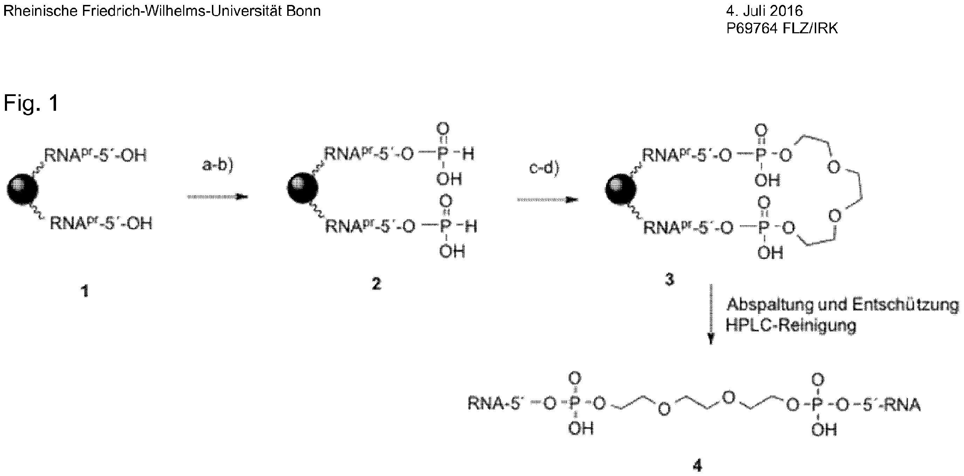

**Figur 1** zeigt ein Reaktionsschema für die Synthese von RNA-5'-X4-5'-RNA (4, X4 = Triethylenglycol-Linker). a) 50 mM 2-Chloro-4H-1,3,2-benzodioxaphosphorin-4-on in Dioxan/Pyridin (3:1 v/v), 30 min; b) 1 M Triethylammoniumbicarbonat, 30 min; c) 0,1 M Pivaloylchlorid, 2 mM Triethylenglykol in Acetonitril/Pyridin (1:1 v/v), 5 h d) 0,1 M lod-Lösung in THF/Pyridin/Wasser, 10 min.

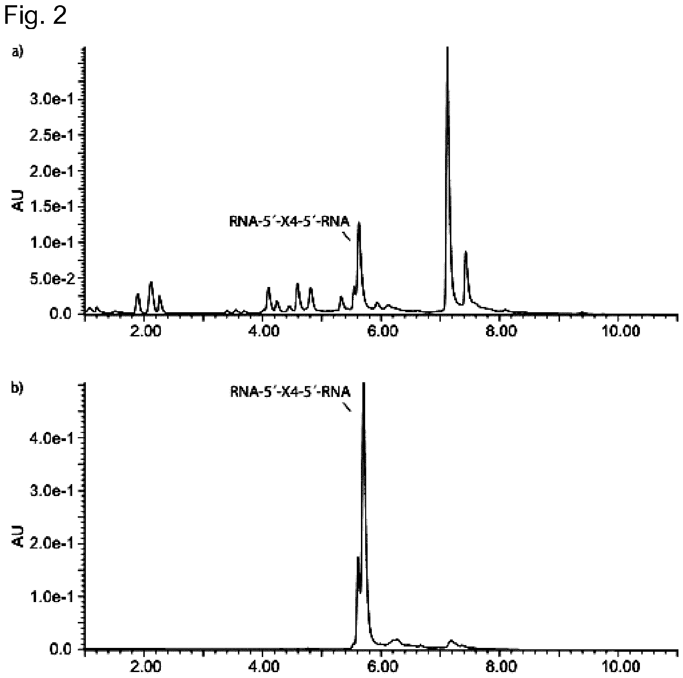

**Figur 2** zeigt RP-LC/MS-Analysen einer RNA-5'-X4-5'-RNA-Synthese (Sequenz: GACGCUGACCCUGAAGUUCAUCUU, X4 = Triethylenglykol-Linker, GFP2_s2n55_T3EG) vor (a) und nach (b) Aufreinigung. Dargestellt ist das UV-Profil der Analyse einer RNA-5'-X4-5'-RNA-Synthese bei Auftrennung an einer Acquity UPLC OST-Säule (Waters, C18, 1,7 µm, 2,1x55 mm). Gradient: 7 % B für 1,5 min, 7-15 % B in 4,75 min, 15-40 % B in 4,5 min, Puffer A: 16,6 mM Triethylamin, 100 mM HFIP, 10 % Methanol, Puffer B: 16,6 mM Triethylamin, 100 mM HFIP, 95 % Methanol. ESI-basierte Massenbestimmung der relevanten Signale: a) 5'-OH-RNA (RT 4,10 min, 3,65 %, calcd MW: 7590 Da, found MW: 7587 Da); 5'-H-p-RNA (RT 4,24 min, 1,60 %, calcd MW: 7653 Da, found MW: 7652 Da); 5'-OH-X4-RNA (RT 4,59 min, 0,33 %, calcd MW: 7802 Da, found MW: 7800 Da); n.d. (RT 4,81 min, 3,8 %, found MW: 7787 Da); RNA-5'-5'-RNA (RT 5,54 min, 2,66 %, calcd MW: Da, found MW: 15238 Da); RNA-5'-X4-5'-RNA (RT 5,62 min, 13,6 %, calcd MW: 15454 Da, found MW: 15450 Da); 5'-Piv-X4-RNA (RT 7,12 min, 35,4 %, calcd MW: 7886 Da, found MW: 7883 Da). b) RNA-5'-5'-RNA (RT 5,61, 14,8 %, calcd MW: Da, found MW: 15238 Da); RNA-5'-X4-5'-RNA (RT 5,70 min, 63,3 %, calcd MW: 15454 Da, found MW: 15450 Da).

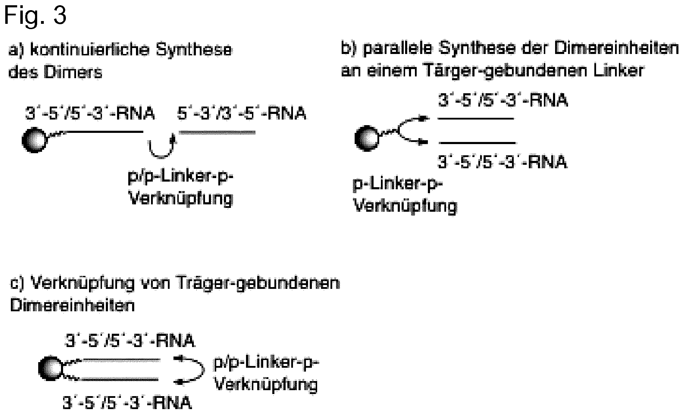

**Figur 3** zeigt eine schematische Darstellung der Synthesestrategien für diskontinuierliche 5'-5'-, 3'-3'- und 3'-5'-verknüpfte RNA-Dimerstrukturen.

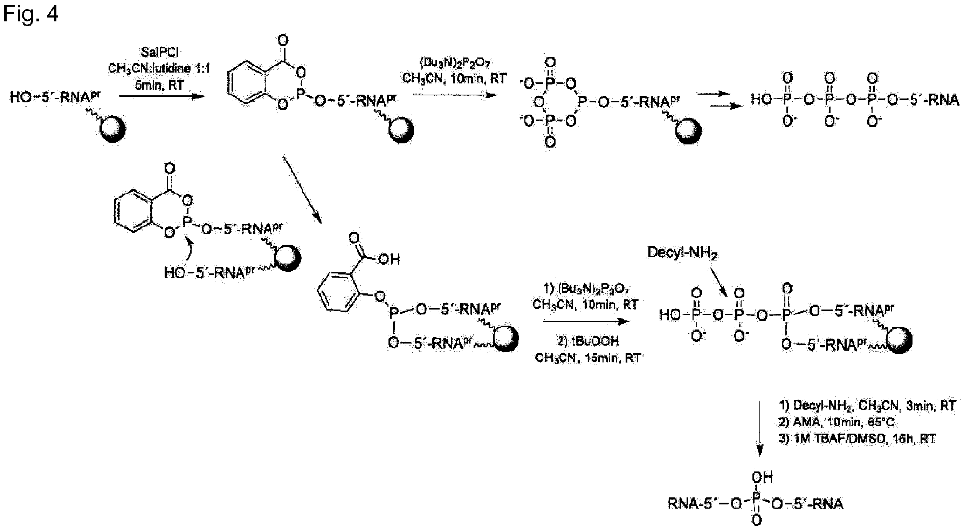

**Figur 4** zeigt beispielhaft die Entstehung von 5'-5'-verknüpften RNA-Dimeren erhalten aus der der 5'-Triphosphat-RNA-Synthese.
**Figur 5** zeigt eine schematische Darstellung eines beispielhaften Verfahrens zur Herstellung von 5'-5'- bzw.3'-3'- symmetrisch verknüpfen dimeren Oligonukleotid-Konjugaten K, wobei B den bivalenten Linker darstellt, der, wie hier beispielhaft dargestellt, ein Phosphatlinker ist. Zunächst liegen die Vorläufer von RNA1 und RNA2 festphasengebunden so vor, dass der von der Festphase abgewandte Terminus geschützt ist (1). Dann wird etwa die Hälfte der Vorläufer terminal entschützt (hier: RNA1), während die andere Hälfte geschützt bleibt (hier: RNA2) (2), In einem weiteren Schritt wird der bivalente Linker B monovalent an den zuvor entschützten Terminus gebunden (hier -OH₂PO₂) (3). Es folgt die Entschützung des anderen festphasengebundenen Vorläuferstrangs (hier: RNA2) (4). Hernach erfolgt die Verknüpfung der beiden Vorläuferstränge (hier: RNA1 und RNA2 über den bivalenten Linker B) zu dem Konjugat RNA1-B-RNA2 (hier: RNA1-O-PO-O-RNA2) (5). Es folgt hier beispielhaft die Oxidation zu einem Phoshorsäurediester, daher RNA1-O-P(O)(OH)-O-RNA2 (6), gefolgt von der Abspaltung des Konjugats (7) und der Anlagerung mit den komplementären Oligonukleotid-Strängen RNA3 und RNA4, die, wie hier dargestellt, an einem Terminus (hier: am 5'-Terminus) triphosphoryliert (ppp) sein können (8), wodurch ein Oligonukleotid-Konjugat K der vorliegenden Erfindung erhalten wird.
**Figur 6** zeigt eine schematische Darstellung eines beispielhaften Verfahrens zur Herstellung von 5'-5'- bzw.3'-3'- symmetrisch verknüpfen dimeren Oligonukleotid-Konjugaten K, wobei B den bivalenten Linker darstellt, der, wie hier beispielhaft dargestellt, ein über zusätzliche, nicht-nukleotide Linkerstrukturen L1 und L2 gebundener Phosphatlinker ist. Das in dieser Figur dargestellte Verfahren läuft ab wie das Verfahren aus Figur 5, nur, dass zusätzliche Linkerstrukturen L1 und L2 zwischen RNA1 und RNA2 und dem Phosphatlinker vorliegen, die ebenso Teil des bivalenten Linkers B werden, der letztlich aus L1, Phosphat und L2 besteht.

### Beispiele

### Material und Methoden

### Reagenzien

Die verwendeten Reagenzien wurden von Sigma-Aldrich oder Roth bezogen und ohne weitere Reinigung eingesetzt.

### RIG-I Stimulationsassay in humanen peripheren mononukleären Zellen (PBMCs)

Für Stimulationsversuche mit humanen PBMCs wurden die Zellen direkt vor Versuchsbeginn isoliert und ausgebracht. Die Zellen wurden mit Chloroquin vorbehandelt, um eine Stimulation der endosomalen TLR-Rezeptoren zu vermeiden und eine selektive Analyse der RIG-I-Aktivität zu ermöglichen. Anhand der Stimulation mit GFP2_as (Einzelstrang-RNA) als TLR-Ligand wurde dabei die Suppression der endosomalen Rezeptoren kontrolliert. Für die Analyse der doppelsträngigen Oligonukleotide wurden die komplementären Einzelstränge zunächst durch zweiminütiges Erwärmen auf 72 °C und langsames Abkühlen auf Raumtemperatur hybridisiert. Anschließend wurden die Stimuli unter Verwendung von Lipofectamin 2000 (Invitrogen) in den angegebenen Konzentrationsreihen (die molaren Konzentrationen beziehen sich jeweils auf die Menge an 24mer Einheiten) in die Zellen eingebracht. Nach 20 h wurde die IFNα-Sekretion mittels ELISA ermittelt. Die Stimulationsassays wurden jeweils in Doppelwertbestimmung für 2-4 Spender durchgeführt.

### Oligonukleotide

5'-OH-Oligoribonukleotid-Synthesen wurden wahlweise von Biomers.net GmbH und Axolabs GmbH bezogen. Die Synthesen von 5'-3'-verknüpften Oligonukleotiden erfolgten unter Verwendung von DMT-2'-O-TBDMS-rC(ac), DMT-2'-O-TBDMS-rA(bz), DMT-2'-O-TBDMS-rG(ib) und DMT-2'-O-TBDMS-rU-Phosphoramiditen im 0.2-1 µmol-Maßstab mit einer CPG-Beladung von 39-44 µmol/g.

Die Einführung von Tetraethylenglykol- und Propyl-Linkern erfolgte unter Verwendung der entsprechenden Phosphoramidite. Für die lineare Synthese von 5'-5'- oder 3'-3'-verknüpften Strukturen wurden Teilsequenzen mit Hilfe der reverse-3'-DMT-rN-5'-CED-Phosphoramidite (ChemGenes Corporation) synthetisiert.

Die parallele Synthese von 5'-5'- oder 3'-3'-verknüpften Strukturen erfolgte an *branching*-Trägern (ChemGenes Cooperation), die über eine Glyceroleinheit mit zwei DMT-geschützten Hydroxylgruppen für die Kettenverlängerung verfügen. Für 5'-triphosphorylierte Sequenzen erfolgte die Triphosphorylierung und Aufreinigung wie zuvor beschrieben (WO2012/130886, Goldeck et al., 2014, Angew. Chem. 126:4782-4786).

**Tabelle 1. Sequenzen einiger verwendeter Oligonukleotide und struktureller Einheiten der Olignukleotid-Konjugate K**

| **Nr.** | **basierend auf SEQ ID NO** | **Bezeichnung** | **Sequenz** |
|---|---|---|---|
| 1 | 1 | GFP2_s | 5'-GACGCUGACCCUGAAGUUCAUCUU-3' |
| 2 | 2 | GFP2_as | 5'-AAGAUGAACUUCAGGGUCAGCGUC-3' |
| 3 | 1^{p} | ppp-GFP2_s | ppp-5'- GACGCUGACCCUGAAGUUCAUCUU-3' |
| 4 | 2^{p} | ppp-GFP2_as | ppp-5'- AAGAUGAACUUCAGGGUCAGCGUC-3' |
| 5 | 1^{#} | GFP2_s2n55 | 3'-UUCUACUUGAAGUCCCAGUCGCAG-5'- 5'-GACGCUGACCCUGAAGUUCAUCUU |
| 6 | 3 | GFP2_s2n53 | 5'-GACGCUGACCCUGAAGUUCAUCUU GACGCUGACCCUGAAGUUCAUCUU-3' |
| 7 | 2⁺ | GFP2_as2n33 | 5'-AAGAUGAACUUCAGGGUCAGCGUC-3'- 3'-CUGCGACUGGGACUUCAAGUAGAA-5' |
| 8 | 1^{M1p} | ppp-GFP2_sOMe1 | ppp-5'-gACGCUGACCCUGAAGUUCAUCUU-3' |
| 9 | 1^{M1} | GFP2_sOMe1 | 5'-gACGCUGACCCUGAAGUUCAUCUU-3' |
| 10 | 2^{M1} | GFP2_asOMe1 | 5'-aAGAUGAACUUCAGGGUCAGCGUC-3' |
| 11 | 1^{M2} | GFP2_sOMe2 | 5'-GaCGCUGACCCUGAAGUUCAUCUU-3' |
| 12 | 1^{M3} | GFP2_sOMe3 | 5'-GAcGCUGACCCUGAAGUUCAUCUU-3' |
| 13 | 1^{M4} | GFP2_sOMe4 | 5'-GACgCUGACCCUGAAGUUCAUCUU-3' |
| 14 | 1^{M5} | GFP2_sOMe5 | 5'-GACGcUGACCCUGAAGUUCAUCUU-3' |
| 15 | 1^{M6} | GFP2_sOMe6 | 5'-GACGCuGACCCUGAAGUUCAUCUU-3' |
| 16 | 1^{M7} | GFP2_sOMe7 | 5'-GACGCUgACCCUGAAGUUCAUCUU-3' |
| 17 | 1^{M8} | GFP2_sOMe8 | 5'-GACGCUGaCCCUGAAGUUCAUCUU-3' |
| 18 | 1^{M9} | GFP2_sOMe9 | 5'-GACGCUGAcCCUGAAGUUCAUCUU-3' |
| 19 | 1^{M10} | GFP2_sOMe10 | 5'-GACGCUGACcCUGAAGUUCAUCUU-3' |
| 20 | 2^{FAM} | GFP2_as5'-FAM | 5 5’-FAM- |
| | | | AAGAUGAACUUCAGGGUCAGCGUC-3' |
| 21 | 2^{#} | GFP2_as2n55 | 3'-CUGCGACUGGGACUUCAAGUAGAA-5'- 5'- AAGAUGAACUUCAGGGUCAGCGUC-3' |
| 22 | 2^{#L} | GFP2_as2n55_C3br | 3'-CUGCGACUGGGACUUCAAGUAGAA-5'- X1-5'- AAGAUGAACUUCAGGGUCAGCGUC-3' |
| 23 | 2^{#L} | GFP2_as2n55_T4EG | 3’-CUGCGACUGGGACUUCAAGUAGAA-5’- X2-5’- AAGAUGAACUUCAGGGUCAGCGUC-3’ |
| 24 | 2^{+L} | GFP2_as2n33_C3br | 5'-AAGAUGAACUUCAGGGUCAGCGUC-3'- X1-3'-CUGCGACUGGGACUUCAAGUAGAA- 5' |
| 25 | 2∼^{L} | GFP2_as2n53_C3 | 5'-AAGAUGAACUUCAGGGUCAGCGUC-3'- X3-5'-AAGAUGAACUUCAGGGUCAGCGUC- 3' |
| 26 | 1^{#L} | GFP2_s2n55_T3EG | 3'-UUCUACUUGAAGUCCCAGUCGCAG-5'- X4-5'-GACGCUGACCCUGAAGUUCAUCUU |

| | | | |
|---|---|---|---|
| ^{p} mit triphosphoryliertem 5'-Terminus # zweimal diese Sequenz über eine 5'-5'-Verknüpfung miteinander verknüpft ⁺ zweimal diese Sequenz über eine 3'-3'-Verknüpfung miteinander verknüpft ^{∼} zweimal diese Sequenz über einen Propyl-Linker 3'-5'-verknüpft ^{M1} wobei das Nukleotid an Position 1 eine 2'-O-Methylierung aufweist ^{M2} wobei das Nukleotid an Position 2 eine 2'-O-Methylierung aufweist ^{M3} wobei das Nukleotid an Position 3 eine 2'-O-Methylierung aufweist ^{M4} wobei das Nukleotid an Position 4 eine 2'-O-Methylierung aufweist ^{M5} wobei das Nukleotid an Position 5 eine 2'-O-Methylierung aufweist ^{M6} wobei das Nukleotid an Position 6 eine 2'-O-Methylierung aufweist ^{M7} wobei das Nukleotid an Position 7 eine 2'-O-Methylierung aufweist ^{M8} wobei das Nukleotid an Position 8 eine 2'-O-Methylierung aufweist ^{M9} wobei das Nukleotid an Position 9 eine 2'-O-Methylierung aufweist ^{M10} wobei das Nukleotid an Position 10 eine 2'-O-Methylierung aufweist ^{FAM} 5'-terminal modifiziert mit FAM (= 6-Carboxyfluorescein) ^{L} verknüpft über einen bivalenten Linker B: X1 = Glykol-Linker, X2 = Tetraethylengylkol-Linker, X3 = Propyl-Linker, X4 = Triethylenglykol-Linker | | | |

### Resultate

### Die Verknüpfung von Oligoribonukleotid-Duplexen über 5'-5'- oder 3'-3'-Phosphodiesterbindungen führt zu hoch aktiven RIG-I Liganden

Es ist bekannt, dass 5'-Triphosphat-dsRNAs als potente Aktivatoren des Immunsensors RIG-I dienen. Im Vergleich dazu wurde nun untersucht, ob neben der Triphosphat-Gruppe auch strukturelle Modifikationen eine Modulation der RIG-I-Ligandeigenschaften von 5'-OH-RNAs ermöglichen. In einem ersten Beispiel wurde die Verknüpfung von zwei 5'-OH-dsRNA 24mer-Einheiten zu Dimeren über 5'-5'-, 3'-3'- und 3'-5'-Phosphodiesterbindungen vergleichend untersucht.
Als Modellsequenz für die folgenden Untersuchungen wurde die 24mer 'GFP2'-Sequenz gewählt. 48mer-Strukturen wurden über Standard-RNA-Synthesen erhalten, wobei für die Einführung der 5'-5'- und 3'-3'-Verknüpfungen jeweils eine Teilsequenz unter Verwendung der *reverse* RNA Amidite (ChemGenes Corporation) aufgebaut wurde. Anschließend wurden die Sequenzen mit den komplementären 24mer Gegensträngen ergänzt und in humanen PBMCs auf RIG-I-Aktivität überprüft (Tabelle 2 und 3). Für das 5'-3'-verknüpfte Dimer wurde wie für 5'-OH-dsRNAs erwartet eine sehr schwache RIG-I-Aktivität beobachtet. Im Vergleich dazu führte überraschenderweise die Verknüpfung der zwei 24mer-Einheiten über 5'-5'- oder 3'-3'-Phosphodiesterbindungen zu hoch aktiven RIG-I-Liganden. Somit führen diese Ergebnisse die 5'-5'- oder 3'-3'-Verknüpfung von dsRNA-Einheiten als neues RIG-I-aktivitätssteigerndes Strukturelement ein.

**Tabelle 2. Vergleich der RIG-I-Aktivität von 5'-5'- und 5'-3'-verknüpften 5'-OH-dsRNA-Dimeren.**

| RNA (nM) | IFNα (ng/ mL) | | | | | | | | | |
|---|---|---|---|---|---|---|---|---|---|---|
| | GFP2_as (Einzelstrang, Seq. Nr. 2) | | Medium | | ppp-GFP2 (Seq. Nr. 3/2) | | GFP2_s2n55 (Seq. Nr. 5/2) | | GFP2_s2n35 (Seq. Nr. 6/2) | |
| | MW | Stdabw | MW | Stabw | MW | Stabw | MW | Stabw | MW | Stabw |
| 50,00 | 0,017 | 0,00 | 0,017 | 0,00 | 2,19 | 0,553 | 8,681 | 1,342 | 0,752 | 0,06 |
| 5,00 | | | | | 1,761 | 0,107 | 6,366 | 0,295 | 0,718 | 0,036 |
| 0,50 | | | | | 0,078 | 0,012 | 2,444 | 0,269 | 0,13 | 0,012 |

Die Verbindungen wurden in einer Titratrionsreihe (50, 5, 0,5 nM) in Chloroquin-behandelte humane PBMCs eingebracht und die stimulatorische Aktivität wurde über Messung der IFNα-Sekretion nach 20 h erfasst. Die molaren Konzentrationen beziehen sich auf den Gehalt an 24mer-Einheiten. Dargestellt sind Mittelwert und Standardabweichung aus der Doppelwertbestimmung eines repräsentativen Spenders (n=4).

**Tabelle 3. Vergleich der RIG-I-Aktivität von 5'-5'- und 3'-3'-verknüpften 5'-OH-dsRNA Dimeren.**

| RNA (nM) | IFNα (ng/ mL) | | | | | | | | | |
|---|---|---|---|---|---|---|---|---|---|---|
| | GFP2_as (Einzelstrang, Seq. No. 2) | | Medium | | ppp-GFP2 (Seq. No. 3/2) | | GFP2_s2n55 (Seq. No. 5/2) | | GFP2_as2n33 (Seq. No. 7/1) | |
| | MW | Stabw | MW | Stabw | MW | Staw | MW | Stabw | MW | Stabw |
| 17,00 | 0,00 | 0,00 | 0,02 | 0,03 | 0,64 | 0,01 | 1,13 | 0,10 | 0,68 | 0,00 |
| 5,00 | | | | | 0,34 | 0,07 | 1,23 | 0,11 | 0,42 | 0,12 |
| 0,50 | | | | | 0,05 | 0,00 | 0,56 | 0,00 | 0,06 | 0,03 |

Die RNA-Duplexe wurden in einer Titratrionsreihe (17, 5, 0,5 nM) in Chloroquin-behandelte humane PBMCs eingebracht und die stimulatorische Aktivität wurde über Messung der IFNα-Sekretion nach 20 h erfasst. Die molaren Konzentrationen beziehen sich auf den Gehalt an 24mer-Einheiten. Dargestellt sind Mittelwert und Standardabweichung aus der Doppelwertbestimmung eines repräsentativen Spenders (n=4).

### Die Immunerkennung von 5'-5'- und 3'-3'-verknüpften dsRNAs durch RIG-I

Im Folgenden wurden die neuartigen symmetrischen 5'-5'- und 3'-3'-verknüpften Dimerliganden anhand von Struktur-Aktivitätsanalysen näher untersucht, um eine Erkennung durch den Rezeptor RIG-I sicherzustellen und näher zu charakterisieren. Für die Erkennung kurzer RNA-Duplexe durch RIG-I ist allgemein bekannt, dass zunächst das 5'-Ende durch die C-terminale Domäne erkannt wird. Dann erfolgt die Bindung der Helikase-Domäne an den RNA-Duplex, Konformationsänderung und Aktivierung durch Freisetzung der CARD-Domänen, die die weitere Signalweiterleitung vermitteln. Die Bindungsdetails der RIG-I-CTD an 5'-Triphosphat-dsRNA und auch an 5'-OH-dsRNA konnten jeweils anhand von Kristallstrukturen aufgeklärt werden (Wang et al. 2010, Nature Structural & Molecular Biology, Nat Struct Mol Biol. 2010 Jul;17(7);781-787; Lu et al. 2010, Nucleic Acids Res. 2011 Mar;39(4): 1565-1575). In beiden Fällen stellen eine *stacking*-Wechselwirkung des 5'-terminalen Basenpaars mit dem Phenylalaninrest F853 und eine Wasserstoffbrückenbindung zwischen der 2'-Hydroxylgruppe des ersten Nukleotids und dem Histidinrest H830 essentielle Bindungskontakte dar. Für die Erkennung sowohl von 5'-ppp-dsRNA als auch von 5'-OH-dsRNA ist somit neben einer intakten Basenpaarung am 5'-Ende eine freie 2'-OH-Gruppe am terminalen 5'-Nukleotid essentiell. Es wurden daher diese bekannten Bindungskontakte für Substitutionsstudien mit ppp-dsRNA und den neuartigen 5'-5'- und 3'-3'-verknüpften 5'-OH-dsRNA-Dimeren vergleichend untersucht (Tabelle 4).

Es konnte bestätigt werden, dass eine 2'-OMe-Substitution am ersten Nukleotid am 5'-Triphosphat-Terminus von ppp-dsRNA-Liganden (pppGFP2_sOMe1) die RIG-I CTD-Anbindung über sterische Hinderung der Interaktion mit H830 verhindert und die Interferonantwort in hPBMCs unterbindet. Am entgegengesetzen, inneren 5'-OH-Ende von ppp-dsRNA hat eine 2'-OMe-Substitution am terminalen Nukleotid (pppGFP2_asOMe1) nur einen geringfügigen Einfluss auf die stimulatorische Aktivität. Dies bestätigt, dass bei der RIG-I-Erkennung von ppp-dsRNA das der Triphosphat-Gruppe entgegengesetzte Duplexende nicht von der CTD-Domäne gebunden wird. Die Analyse der 2'-OMe-Substitutionseffekte eignete sich somit, um die Duplex-Enderkennung durch RIG-I abzufragen, und es wurden im 5'-5'- und 3'-3'-verknüpften Dimer sowohl der Effekt der Substition der nach außen gerichteten Enden (GFP2_s2n55_asOMe1) als auch eine Methylierung an den 5'-terminalen Nukleotiden der, inneren' Phosphatverknüpften Enden (GFP2_as2n33_sOMe1) untersucht. Ein vollständiges Ausbleiben der Interferonantwort bei Substitution der äußeren Enden und eine weitgehende Toleranz an den inneren Enden lieferte einen eindeutigen Anhaltspunkt, dass die Immunerkennung der Dimerstrukturen ausschließlich durch RIG-I vermittelt wird und die RIG-I-Aktivierung durch die RIG-I CTD-Anbindung an den äußeren 5'-OH-Enden initiiert wird.

Im Rahmen weiterer Substitutionsexperimente an den 5'-terminalen Nukleotiden wurde der Einfluss einer sterischen Hinderung untersucht. Eine Substitution mit 5'-Fluorescein als sperriger Rest an den äußeren Enden (GFP2_s2n55_asFAM) führte dabei zu vollständigem Aktivitätsverlust (Tabelle 4). Andererseits wurde zur Erhöhung der RIG-I-Affinität der äußeren Enden eine 5'-Triphosphatgruppe eingeführt (GFP2_s2n55s_as-ppp). Hierdurch wurde die Aktivität des GFP2-Dimers weiter potenziert und selbst in niedrigen Konzentrationen maximale IFNα-Mengen gemessen (Tabelle 5). Diese Beobachtungen unterstützen somit weiter die Hypothese der Notwendigkeit einer RIG-I CTD-Anbindung an den äußeren Dimer-Enden für die stimulatorische Aktivität.

Nach diesen Untersuchungen zum ersten Bindungskontakt zwischen der RIG-I CTD-Domäne und dem 5'-Terminus der Liganden wurde im nächsten Schritt die weitere RIG-I-Aktivierung mit Bindung der Helikase-Domäne durch Substitutionsanalysen an den ersten 10 Nukleotiden untersucht. Es wurden dabei vergleichend die 2'-O-Methylierung im 5'-OH-dsRNA 24mer -und im 5'-5'-verknüpften 48mer-Liganden analysiert, wobei im Falle des Dimers eine Substitution an den äußeren Duplexenden erfolgte (Tabelle 6 und 7). Im Einklang mit den bisherigen Beobachtungen zeigten die 24mere dabei allgemein weit geringere Aktivitäten als die entsprechenden Dimerverbindungen. Für beide Verbindungen konnte übereinstimmend eine relative Abnahme der Interferonantwort bei 2'-O-Methylierungen an den Nukleotiden 4-9 beobachtet werden. Diese Ergebnisse untermauern einen gemeinsamen Erkennungsmechanismus der 5'-OH-dsRNA 24mer-Verbindungen und der 5'-5'-verknüpften Dimerverbindungen durch RIG-I mit vergleichbaren Bindungskontakten mit der RIG-I CTD-Domäne und auch mit der Duplexerkennenden Helikase-Domäne hin.

**Tabelle 4. Substitutionseffekte der 2'-O-Methylierung an den terminalen Nukleotiden.**

| RNA (nM) | IFNα (ng/ mL) | | | | | | | | | | | | | | | | | |
|---|---|---|---|---|---|---|---|---|---|---|---|---|---|---|---|---|---|---|
| | GFP2_as (Einzelstran g, Seq. Nr. 2) | | Medium | | pppGFP2 (Seq. Nr. 3/2) | | pppGFP2 _asOMe1 (Seq. Nr. 3/10) | | pppGFP2 _sOMe1 (Seq. Nr. 8/2) | | GFP2 _s2n55 (Seq. Nr. 5/2) | | GFP2_s2n 55 _asOMe1 (Seq. Nr. 5/10) | | GFP2 _as2n33 (Seq. Nr. 1/7) | | GFP2 _as2n33_s OMe1 (Seq. Nr. 9/7) | |
| | MW | Stab w | MW | Sta bw | MW | Stab w | MW | Sta bw | MW | Stab w | MW | Sta bw | MW | Sta bw | MW | Sta bw | MW | Sta bw |
| 17,00 | 0,00 | 0,00 | 0,00 | 0,00 | 3,36 | 0,15 | 1,43 | 0,12 | 0,06 | 0,03 | 3,25 | 0,29 | 0,00 | 0,00 | 3,14 | 0,18 | 2,46 | 0,20 |
| 5,00 | | | | | 1,49 | 0,22 | 0,45 | 0,17 | 0,03 | 0,04 | 2,82 | 0,21 | 0,00 | 0,00 | 2,38 | 0,10 | 1,52 | 0,00 |
| 1,70 | | | | | 0,01 | 0,01 | 0,00 | 0,00 | 0,01 | 0,01 | 0,39 | 0,55 | 0,00 | 0,00 | 0,01 | 0,01 | 0,03 | 0,04 |

Es wurden 2'-OMe-Substitutionen an den 5'-terminalen Nukleotiden des 5'-Triphosphat-dsRNA-Liganden pppGFP2 und der 5'-5'- und 3'-3'-verknüpften Duplexe GFP2_s2n55 und GFP2_as2n33 eingeführt und die stimulatorische Aktivität in einer Titrationsreihe in Chloroquin-behandelten PBMCs eingebracht. Die IFNα-Produktion wurde nach 20 h mittels ELISA bestimmt. Die molaren Konzentrationen beziehen sich auf den Gehalt an 24mer-Einheiten. Abgebildet sind jeweils Mittelwerte und Standardabweichung aus der Doppelwertbestimmung eines repräsentativen Spenders (n=4).

**Tabelle 5a. Einfluss der sterischen Hinderung an den äußeren 5'-terminalen Nukleotiden von 5'-5'-verknüpften Dimerliganden auf die RIG-I-Aktivität.**

| RNA (nM) | IFNα (ng/ mL) | | | | | | | |
|---|---|---|---|---|---|---|---|---|
| | GFP2_as (Einzelstrang, Seq. Nr. 2) | | Medium | | ppp-GFP2 (Seq. Nr. 3/2) | | GFP2_s2n55_asFAM (Seq. Nr. 5/20) | |
| | MW | Stabw | MW | Stabw | MW | Stabw | MW | Stabw |
| 50,00 | 0,07 | 0,02 | 0,10 | 0,03 | 20,62 | 2,57 | 0,12 | 0,02 |
| 5,00 | | | | | 9,57 | 0,27 | 0,09 | 0,04 |
| 0,50 | | | | | 0,42 | 0,17 | 0,04 | 0,00 |

Durch die Hybridisierung des 5'-5'-verknüpften Dimers GFP2_s2n55 mit einem 5'-Fluorescein-substituierten komplementären Gegenstrang wurde eine an den äußeren Dimerenden sterisch gehinderte Ligandvariante erhalten. Die RIG-I-Aktivität wurde in Chloroquin-behandelten PBMCs durch Bestimmung der IFNα-Produktion erfasst. Die molaren Konzentrationen beziehen sich auf den Gehalt an 24mer-Einheiten. Abgebildet sind jeweils Mittelwerte und Standardabweichung aus der Doppelwertbestimmung eines repräsentativen Spenders (n=4).

**Tabelle 5b. Einfluss der 5'-Triphosphorylierung an den äußeren 5'-terminalen Nukleotiden von 5'-5'-verknüften Dimerliganden auf die RIG-I-Aktivität.**

| RNA (nM) | IFNα (ng/ mL) | | | | | | | | | |
|---|---|---|---|---|---|---|---|---|---|---|
| | GFP2_as (Einzelstrang, Seq. Nr. 2) | | Medium | | ppp-GFP2 (Seq. Nr. 3/2) | | GFP2_s2n55 (Seq. Nr. 5/2) | | GFP2_s2n55_as-ppp (Seq. Nr. 5/4) | |
| | MW | Stabw | MW | Stabw | MW | Stabw | MW | Stabw | MW | Stabw |
| 50,00 | 0,11 | 0,01 | 0,05 | 0,01 | 28,16 | 3,11 | 24,44 | 8,93 | 36,52 | 2,29 |
| 5,00 | | | | | 3,76 | 0,02 | 13,06 | 1,75 | 35,83 | 1,54 |
| 0,50 | | | | | 0,07 | 0,00 | 0,13 | 0,01 | 29,85 | 1,43 |

Das 5'-5'-verknüpfte Dimer GFP2_s2n55 wurde mit einem 5'-triphosphorylierten komplementären Gegenstrang hybridisiert und in Chloroquin-behandelte PBMCs eingebracht. Nach 20 h wurde die IFNα-Produktion erfasst. Die molaren Konzentrationen beziehen sich auf den Gehalt an 24mer-Einheiten. Abgebildet sind jeweils Mittelwerte und Standardabweichung aus der Doppelwertbestimmung eines repräsentativen Spenders (n=4).

**Tabelle 6. 2'-O-Methylsubstitutionseffekte an den Nukleotiden 1-10 bei der RIG-I-Erkennung von 5'-OH-dsRNA-Liganden.**

| RNA (nM) | IFNα (ng/ mL) | | | | | | | | | | | | | |
|---|---|---|---|---|---|---|---|---|---|---|---|---|---|---|
| | GFP2_as (Einzelstrang, Seq. Nr. 2) | | Medium | | GFP2 (Seq. Nr. 1/2) | | GFP2_sOMe1 (Seq. Nr. 9/2) | | GFP2_sOMe2 (Seq. Nr. 11/2) | | GFP2_sOMe3 (Seq. Nr. 12/2) | | GFP2_sOMe4 (Seq. Nr. 13/2) | |
| | MW | Stabw | MW | Stabw | MW | Stabw | MW | Stabw | MW | Stabw | MW | Stabw | MW | Stabw |
| 50,00 | 0,00 | 0,00 | 0,00 | 0,00 | 6,61 | 1,15 | 0,25 | 0,09 | 0,61 | 0,18 | 4,75 | 0,35 | 0,97 | 0,25 |
| 5,00 | | | | | 0,08 | 0,02 | 0,00 | 0,00 | 0,00 | 0,00 | 0,08 | 0,02 | 0,00 | 0,00 |
| | | | | | | | | | | | | | | |

| IFNα (ng/ mL) | | | | | | | | | | | | | | |
|---|---|---|---|---|---|---|---|---|---|---|---|---|---|---|
| GFP2_sOMe5 (Seq. Nr. 14/2) | | GFP2_sOMe6 (Seq. Nr. 15/2) | | | GFP2_sOMe7 (Seq. Nr. 16/2) | | GFP2_sOMe8 (Seq. Nr. 17/2) | | GFP2_sOMe9 (Seq. Nr. 18/2) | | GFP2_sOMe10 (Seq. Nr. 19/2) | | | |
| MW | Stabw | MW | Stabw | MW | Stabw | | MW | Stabw | MW | Stabw | MW | Stabw | | |
| 1,04 | 0,05 | 0,47 | 0,02 | 0,22 | 0,04 | | 0,83 | 0,10 | 1,08 | 0,15 | 4,75 | 0,24 | | |
| 0,00 | 0,00 | 0,00 | 0,00 | 0,02 | 0,02 | | 0,00 | 0,00 | 0,00 | 0,00 | 0,02 | 0,02 | | |

Es wurden 5'-OH-GFP2-Verbindungen mit 2'-O-Methylierungen an den Nukleotiden 1-10 mit dem komplementären 24mer Gegenstrang hybridisiert und zur Stimulation von Chloroquin-behandelten humanen PBMCs verwendet. Die Transfektion erfolgte mit 50 und 5nM RNA-Konzentrationen (bezogen auf die Menge an 24mer Einheiten) und nach 20 h wurden die IFNα-Mengen im Überstand per ELISA quantifiziert. Abgebildet sind Mittelwert und Standardabweichung aus der Doppelwertbestimmung eines repräsentativen Spenders (n=2).

**Tabelle 7. 2'-O-Methylsubstitutionseffekte an den Nukleotiden 1-10 bei der RIG-I-Erkennung von 3'-3'-verknüften 5'-OH-dsRNA-Dimerlianden.**

| RN A (n M) | IFNα (ng/ mL) | | | | | | | | | | | | | | | | | | | | |
|---|---|---|---|---|---|---|---|---|---|---|---|---|---|---|---|---|---|---|---|---|---|
| | GFP2_as (Einzelstr ang, Seq. Nr. 2) | | | | Medium | | GFP2_as 2n55 (Seq. Nr. 1/21 | | | GFP2_as 2n55_sO Me1 (Seq. Nr. 9/21) | | | GFP2_as 2n55_sO Me2 (Seq. Nr. 11/21) | | GFP2_as 2n55_sO Me3 (Seq. Nr. 12/21) | | | | GFP2_as 2n55_sO Me4 (Seq. Nr. 13/21) | | |
| | MW | | Sta bw | | MW | Sta bw | MW | Sta bw | | MW | | Sta bw | MW | Sta bw | MW | | Sta bw | | MW | | Sta bw |
| 50, 00 | 0,00 | | 0,00 | | 0,00 | 0,00 | 15,3 3 | 9,45 | | 0,00 | | 0,00 | 13,0 2 | 2,90 | 22,5 4 | | 8,59 | | 17,2 6 | | 3,19 |
| 5,0 0 | | | | | | | 11,3 2 | 1,87 | | 0,00 | | 0,00 | 6,13 | 0,87 | 24,1 7 | | 0,39 | | 8,95 | | 0,42 |
| | | | | | | | | | | | | | | | | | | | | | |

| IFNα (ng/ mL) | | | | | | | | | | | | | | | | | | | | | |
|---|---|---|---|---|---|---|---|---|---|---|---|---|---|---|---|---|---|---|---|---|---|
| GFP2_as2n5 5_sOMe5 (Seq. Nr. 14/21) | | | | GFP2_as2n5 5_sOMe6 (Seq. Nr. 15/21) | | | GFP2_as2 n55_sOMe 7 (Seq. Nr. 16/21) | | | | GFP2_as2n5 5_sOMe8 (Seq. Nr. 17/21) | | | GFP2_as2n5 5_sOMe9 (Seq. Nr. 18/21) | | | | GFP2_as2 n55_sOMe 10 (Seq. Nr. 19/21) | | | |
| MW | | Stab w | | MW | | Stab w | MW | | Stab w | | MW | | Stab w | MW | | Stab w | | MW | | Stab w | |
| 11,88 | | 3,04 | | 13,44 | | 0,25 | 8,61 | | 1,01 | | 12,26 | | 1,62 | 14,51 | | 0,20 | | 19,0 6 | | 1,82 | |
| 8,16 | | 0,37 | | 10,76 | | 0,30 | 4,13 | | 0,27 | | 7,94 | | 0,22 | 15,49 | | 1,67 | | 21,0 1 | | 2,80 | |

Es wurden 5'-OH-GFP2-Verbindungen mit 2'-O-Methylierungen an den Nukleotiden 1-10 mit dem komplementären 5'-5'-verknüpften 48mer Gegenstrang hybridisiert und zur Stimulation von Chloroquin-behandelten humanen PBMCs verwendet. Die Transfektion erfolgte mit 50 und 5nM RNA-Konzentrationen (bezogen auf die Menge an 24mer Einheiten) und nach 20 h wurden die IFNα-Mengen im Überstand per ELISA quantifiziert. Abgebildet sind Mittelwert und Standardabweichung aus der Doppelwertbestimmung eines repräsentativen Spenders (n=2).

### Variation der RNA-Sequenz bei der RNA-5'-p-5'-RNA-Erkennung

In den ersten Beispielen wurde die Dimerbildung der GFP2-Modellsequenz am sonst triphosphorylierten 5'-Terminus (5'-GACG..., GFP2_s2n55 und GFP2_as2n33) untersucht, bei der die entgegengesetzten Enden (5'-AAGA...) im Dimer nach außen gerichtet sind und somit die Erkennung durch RIG-I vermitteln. Als erste Sequenzvariation wurden durch die alternative Verknüpfung der GFP2-Duplexe über die 5'-Enden des antisense-Strangs (GFP2_2n55as) nun die entgegengesetzten 5'-Termini (5'-GACG...) nach außen gerichtet. Bei Analyse der RIG-I-Aktivität in humanen PBMCs konnte analog zu den Triphosphat-Verbindungen unabhängig von der Sequenz für die Dimerverbindungen eine gleichermaßen starke Interferonantwort beobachtet werden (Tabelle 8). Auch für dieses Dimer ließ sich durch eine zusätzliche Triphosphorylierung der äußeren Enden (pppGFP2_as2n55) die RIG-I-Aktivität weiter potenzieren. Diese Ergebnisse lassen auf eine allgemeine Anwendbarkeit der aktivitätsfördernden 5'-5'- und 3'-3'-Strukturmotive auf weitere RNA-Dimersequenzen schließen.

**Tabelle 8. Vergleich der Dimerverbindungen GFP2_2n55s und GFP2_2n55as.**

| RNA (nM) | IFNα (ng/ mL) | | | | | | | | | | | | | |
|---|---|---|---|---|---|---|---|---|---|---|---|---|---|---|
| | GFP2_as (Einzelstrang, Seq. Nr. 2) | | ppp-GFP2 | | GFP2_ppp-as (Seq. Nr. 3/2) | | GFP2_s2n55 (Seq. Nr. 5/2) | | GFP2_s2n55_as-ppp (Seq. Nr. 5/4) | | GFP2_as2n55 (Seq. Nr.1/21) | | pppGFP2_as2n55 (Seq. Nr. 3/21) | |
| | MW | Stabw | MW | Stabw | MW | Stabw | MW | Stabw | MW | Stabw | MW | Stabw | MW | Stabw |
| 5,00 | 0,16 | 0,00 | 14,08 | 1,00 | 17,88 | 1,85 | 19,10 | 1,11 | 22,45 | 0,54 | 24,86 | 2,58 | 25,73 | 1,50 |
| 1,60 | | | 9,73 | 0,53 | 13,18 | 0,19 | 17,31 | 1,50 | 24,35 | 1,54 | 19,05 | 0,19 | 25,05 | 1,46 |
| 0,50 | | | 4,04 | 0,45 | 2,13 | 0,91 | 7,44 | 0,42 | 19,76 | 2,81 | 6,94 | 4,40 | 20,76 | 2,92 |
| 0,17 | | | 0,27 | 0,06 | 0,20 | 0,06 | 0,41 | 0,14 | 11,47 | 1,08 | 0,65 | 0,37 | 14,59 | 3,81 |
| 0,05 | | | 0,16 | 0,00 | 0,16 | 0,00 | 0,12 | 0,06 | 8,74 | 0,82 | 0,12 | 0,06 | 9,88 | 2,01 |
| 0,01 | | | 0,08 | 0,11 | 0,20 | 0,17 | 0,20 | 0,06 | 5,39 | 0,48 | 0,04 | 0,06 | 5,68 | 0,24 |

Die Dimerverbindungen GFP2_s2n55 und GFP2_as2n55 wurden mit dem komplementären OH-RNA- und dem pppRNA-Gegenstrang hybridisiert und im Vergleich zu pppGFP2 und GFP2_as-ppp zur Stimulation von Chloroquin-behandelten humanen PBMCs verwendet. Die Transfektion erfolgte als Titrationsreihe (5, 1.6, 0,5, 0.16, 0.05, 0.005 nM, bezogen auf die Konzentration an 24mer-Einheiten) und nach 20 h wurden die IFNα-Mengen im Überstand per ELISA quantifiziert. Abgebildet sind Mittelwert und Standardabweichung aus der Doppelwertbestimmung eines repräsentativen Spenders (n=2).

### Die Einführung von Linkerstrukturen an der 5'-3', 5'-5'- und 3'-3'-Bindung zwischen den Dimereinheiten wird durch RIG-I toleriert

In der weiteren Untersuchung zu Ligandanforderungen wurde der Einfluss verschiedener Linkerstrukturen an der 5'-5'- bzw. 3'-3'-Verknüpfung der Dimere analysiert, wobei die Verbindungen GFP2_as2n55 und GFP2_as2n33 als Beispielsequenzen dienten. Zunächst wurde die Einführung einer C3-Einheit untersucht. Als Syntheseansatz wurde hier die parallele Synthese der zwei 24mer-Einheiten an einem CPG-gebundenen Glycerol-Linker (*branching* 3'-Icaa-CPG, ChemGenes Corperation) gewählt. Dieses Trägermaterial ermöglicht ausgehend von der Linkereinheit sowohl eine 5'-5'-Verknüpfung unter Verwendung der *reverse* RNA-Amidite für die RNA-Synthese als auch eine 3'-3'-Verknüpfung unter Verwendung der Standard-RNA-Amidite. Als Beispielverbindungen wurden so die Verbindungen GFP2_as2n55_C3br und GFP2_as2n33_C3br synthetisiert. Des Weiteren wurde eine Tetraethylenglykol-Einheit als Beispiel für eine längere Linkerstruktur in die GFP2_as2n55-Verbindung eingefügt (GFP2_as2n55_T4EG). Hier wurde die Synthese als kontinuierliche RNA-Synthese unter Verwendung des entsprechenden Tetraethylenglykol-Phosphoramidits und Einsatz der *reverse* RNA-Amidite realisiert. Neben diesen 5'-5'- und 3'-3'-Verknüpfungen wurde auch der Einfluss der Einführung von Linkerstrukturen im Kontext eines regulären, 5'-3'-verknüpften Dimers untersucht. Als Beispielsynthese wurde hierzu ein Propandiol-Linker während der kontinuierlichen RNA-Synthese eingebracht (GFP2_as2n53_C3). Bei Testung in humanen PBMCs zeigten alle synthetisierten Linker-haltigen Dimerverbindungen eine hohe RIG-I-Aktivität und es wurde eine im Vergleich zu den 5'-5'- und 3'-3'-Phosphat-verknüpften Dimeren eine vergleichbare bis leicht schwächere Interferonantwort erzielt (Tabelle 9 und 10). Die Aktivität der Linker-verknüpften Dimere ließ sich durch eine zusätzliche Einführung von 5'-Triphosphatgruppen weiter steigern. Diese Ergebnisse zeigen, dass die Einführung von Linkerstrukturen in die 5'-5'- und 3'-3'-verknüpften RNA-Dimere unter Erhalt der hohen RIG-I-Aktivität möglich ist und auch die Unterbrechung von kontinuierlichen 5'-3'-verknüpften Dimeren durch Linkerstrukturen eine Aktivitätssteigerung mit sich führen kann. Somit lässt sich das RIG-I-aktivitätsfördernde Strukturelement auf die Gruppe der Linker-überbrückten 5'-3'-, 5'-5'- und 3'-3'-Verknüpfungen ausweiten. Dies ermöglicht eine flexible und vielseitige Synthese der entsprechenden Liganden, wobei neben der kontinuierlichen 48mer-Synthese eine parallele Synthese der 24mer-Einheiten an *branching* CPG-Einheiten als weiterer Syntheseansatz ermöglicht wird.

**Tabelle 9. Die Aktivierung von RIG-I durch 5'-5'-verknüpfte RNA-p-Linker-p-RNA-Verbindungen.**

| RNA (nM) | IFNα (ng/ mL) | | | | | | | | | | | | | | | | | |
|---|---|---|---|---|---|---|---|---|---|---|---|---|---|---|---|---|---|---|
| | GFP2_as (Einzelstran g, Seq. Nr. 2) | | Medium | | ppp-GFP2 (Seq. Nr. 3/2) | | GFP2_as2n 55 (Seq. Nr. 5/21) | | GFP2_as2n5 5_C3br (Seq. Nr. 1/22) | | GFP2_as2n55 _T4EG (Seq. Nr. 1/23) | | pppGFP2_as 2n55 (Seq. Nr. 3/21) | | pppGFP2_as 2n55_C3br (Seq. Nr. 3/22) | | pppGFP2_as 2n55_T4EG (Seq. Nr. 3/23) | |
| | MW | Stab w | MW | Stab w | MW | Stab w | MW | Stab w | MW | Stab w | MW | Stab w | MW | Stab w | MW | Stab w | MW | Stab w |
| 50,00 | 0,02 | 0,03 | 0,00 | 0,00 | 27,93 | 2,07 | 23,81 | 5,00 | 21,80 | 1,78 | 21,80 | 2,07 | 22,38 | 4,33 | 22,38 | 4,33 | 22,52 | 1,35 |
| 5,00 | | | | | 21,50 | 2,02 | 27,52 | 3,42 | 14,32 | 2,93 | 12,27 | 3,72 | 26,02 | 1,40 | 26,02 | 1,40 | 24,66 | 0,72 |
| 0,50 | | | | | 1,70 | 0,57 | 2,78 | 0,90 | 0,16 | 0,07 | 0,07 | 0,05 | 24,49 | 2,21 | 24,49 | 2,21 | 20,99 | 7,26 |
| 0,05 | | | | | 0,04 | 0,00 | 0,02 | 0,03 | 0,00 | 0,00 | 0,02 | 0,03 | 19,69 | 0,53 | 19,69 | 0,53 | 13,16 | 0,91 |

Die über einen Glycol-Linker (C3br), über einen Propan-Linker (C3) oder über einen Tetraethylen-Linker (T4EG) verknüpften GFP2_as2n55 Dimere wurden mit den komplementären 5'-OH- und 5'-Triphosphat-Gegensträngen hybridisiert. Als Referenzsubstanz diente der pppGFP2-Duplex und der Phosphodiester-verknüpfte GFP2_s2n55-Duplex. Die Verbindungen wurden zur Stimulation von Chloroquin-behandelten humanen PBMCs verwendet. Die Transfektion erfolgte in einer Titrationsreihe (50, 5, 0.5, 0.005 nM) und nach 20 h wurden die IFNα-Mengen im Überstand per ELISA quantifiziert. Die angegebenen Konzentrationen beziehen sich auf den Gehalt an 24mer Einheiten. Abgebildet sind Mittelwert und Standardabweichung aus der Doppelwertbestimmung eines repräsentativen Spenders (n=2).

**Tabelle 10. Die Aktivierung von RIG-I durch 3'-3- und 3'-5'-verknüpfte RNA-p-Linker-p-RNA-Verbindungen.**

| RNA (nM) | IFNα (ng/ mL) | | | | | | | | | | | |
|---|---|---|---|---|---|---|---|---|---|---|---|---|
| | GFP2_as (Einzelstra ng, Seq. Nr. 2) | | Medium | | ppp-GFP2 (Seq. Nr. 3/2) | | GFP2_as2 n33 (Seq. Nr. 1/7) | | GFP2_as2 n33_C3br (Seq. Nr. 1/24) | | GFP2_as2 n53_C3 (Seq. Nr. 1/25) | |
| | MW | Stab w | MW | Stab w | MW | Stab w | MW | Stab w | MW | Stab w | MW | Stab w |
| 17,00 | 0,00 | 0,00 | 0,00 | 0,00 | 16,1 5 | 0,95 | 19,6 6 | 1,15 | 15,0 6 | 2,22 | 17,9 2 | 0,08 |
| 5,00 | | | | | 12,9 4 | 1,52 | 12,9 8 | 3,76 | 11,0 0 | 2,96 | 14,6 6 | 4,85 |
| 1,70 | | | | | 9,86 | 1,10 | 5,00 | 0,95 | 5,00 | 0,95 | 4,35 | 0,50 |

Für die 3'-3'- und 3'-5'-Verknüpfung von Dimeren wurde der Einfluss eines Glycol-Linkers (C3br) und eines Propan-Linkers (C3) auf die RIG-I-Aktivität untersucht. Die Dimere wurden mit den komplementären Gegensträngen hybridisiert und zur Stimulation von Chloroquin-behandelten PBMCs verwendet. Nach 20 h wurde die IFNα-Mengen im Überstand per ELISA quantifiziert. Die angegebenen Konzentrationen beziehen sich auf den Gehalt an 24mer Einheiten. Abgebildet sind Mittelwert und Standardabweichung aus der Doppelwertbestimmung eines repräsentativen Spenders (n=2).

### Die Synthese von dimeren RIG-I-Liganden durch Linker-vermittelte 5'-5'-Verknüpfung träger-gebundener RNA-Sequenzen

Neben der parallelen Synthese der Teilsequenzen an *branching*-Trägermaterialien stellt auch die Möglichkeit der Verknüpfung von trägergebundenen 5'-OH-RNA-Sequenzen eine attraktive Synthesestrategie für dimere RIG-I-Liganden dar. Hierzu wurde die Phosphitylierung und Hydrolyse von trägergebunden 5'-OH-RNA-Sequenzen zu den H-Phosphonat-Derivaten gefolgt von der Reaktion mit bifunktionellen Linkern in Anwesenheit von Pivaloylchlorid und Oxidation der Phosphit- zu Phosphatgruppen eingeführt. Dieser Syntheseansatz konnte beispielsweise auf die 5'-5'-Verknüpfung der GFP2-Sequenz über einen Triethylenglykol-Linker (GFP2_2n55_T3EG) erfolgreich angewandt werden (Figur 1 und 2).

Es wurde die vollständig geschützte, trägergebundene 5'-OH-GFP2-Modellsequenz (1 µmol) zunächst mit einer wasserfreien Pyridin/Dioxan-Lösung (1:3, v/v, 4 mL) gewaschen. Anschließend wurde eine frische angesetzte 50 mM 2-Chloro-4H-1,3,2-benzodioxaphosphorin-4-on-Lösung (2 mL, 100 µmol) in trockenem Dioxan/Pyridin (3:1, v/v) mit Hilfe von Plastikspritzen über die Säule geleitet. Die Lösung wurde für eine 30-minütige Reaktionszeit wiederholt über die Plastikspritze durch Anziehen und Ausstoßen mit der Synthesesäule in Kontakt gebracht. Anschließend wurde die Synthesesäule mit Acetonitril (3 mL) gewaschen. Im nächsten Schritt wurde die Synthesesäule mit einer wässrigen 1 M Triethylammoniumbicarbonat-Lösung (1 mL) für 30 min inkubiert und so die trägergebundene 5'-H-Phosphonat-RNA erhalten. Im Anschluss wurde die Synthesesäule mit Acetonitril (3 mL) und Acetonitril/Pyridin (1:1, v/v, 3 mL) gewaschen. Für die Linker-vermittelte Verknüpfung wurde eine Lösung an Pivaloylchlorid (12 µL, 0,1 mmol) und Triethylenglycol (0,27 µL, 2 µmol) in Acetonitril/Pyridin (1:1, v/v, 1 mL) angesetzt und über die Synthesesäule geleitet. Nach einer Inkubationszeit von 5 h erfolgte nach einem weiteren Waschschritt mit Acetonitril (3 mL) die Oxidation mit einer 0,1 M Iod-Lösung in THF/Pyridin/Wasser (2 mL) für 10 min. Anschließend wurde das Syntheseprodukt mit Acetonitril (3x3 mL) gewaschen, im Argonstrom getrocknet und unter Standardbedingungen vom Trägermaterial gespalten und entschützt. Das 5'-5'-verknüpfte Dimerprodukt wurde über HPLC-Chromatographie an einer Anionenaustauschsäule (Source15Q 4.6/100, GE Healthcare) im Natriumperchloratgradienten (20 mM Tris-HCl, pH8, 1 mM EDTA, 80-320 mM NaClO₄) aufgereinigt und über eine HiTrap-Säule (GE Healthcare) entsalzt. Die Integrität des erhaltenen Produkts wurde über RP-LC/MS-Analyse bestätigt.
Die RIG-I-Aktivität des erhaltenen GFP2_s2n55_T3EG-Produkts wurde im Stimulationsexperiment in humanen PBMCs ermittelt. Es wurden im Vergleich zu ppp-GFP2 leicht höhere und mit GFP2_s2n55 vergleichbare Interferon-Antworten gemessen (Figur 3). Somit konnte bestätigt werden, dass auch über Linker verknüpfte 5'-5'-Dimerstrukturen hoch aktive RIG-I-Liganden darstellen.

Zusammenfassend konnten somit für die Synthese von über 5'-5'- und 3'-3'-Phosphodiesterbindungen verknüpfte und über 5'-5'-, 3'-3'- oder 3'-5'-Linkerstrukturen verbundene RIG-I-Ligandstrukturen die kontinuierliche Synthese und die parallele Synthese der Dimereinheiten an Verzweigungseinheiten oder in Kombination mit einer postsynthetischen Verknüpfung aufgezeigt werden (Figur 3). Vor allem die parallele Synthese der kurzen Dimereinheiten kann dabei effizientere und kostengünstigere Syntheseverfahren und die Bereitstellung der Liganden in hoher Reinheit ermöglichen.

**Tabelle 11. Die RIG-I-Aktivität eines über einen Triethylenglykol-Linker verknüpften 5'-5'-Dimers.**

| RNA (nM) | IFNα (ng/ mL) | | | | | | | | | |
|---|---|---|---|---|---|---|---|---|---|---|
| | GFP2_as (Einzelstran g, Seq. No. 2) | | Medium | | ppp-GFP2 (Seq. No. 3/2) | | GFP2_s2n5 5 (Seq. No. 5/2) | | GFP2_s2n5 5_T3EG (Seq. No. 26/2) | |
| | MW | Stab w | MW | Stab w | MW | Stab w | MW | Stab w | MW | Stab w |
| 50,00 | 0,00 | 0,00 | 0,00 | 0,00 | 7,37 | 0,25 | 7,86 | 0,98 | 6,38 | 0,93 |
| 17,00 | | | | | 5,69 | 0,00 | 8,43 | 0,00 | 8,05 | 1,47 |
| 5,00 | | | | | 5,94 | 0,00 | 7,72 | 1,67 | 6,23 | 0,09 |
| 1,70 | | | | | 2,23 | 0,38 | 5,71 | 1,30 | 3,20 | 1,44 |

Das über einen Triethylenglykol-Linker 5'-5'-verknüpfte GFP2_s2n55_T3EG-Dimer wurde mit dem komplementären Gegenstrang hybridisiert und im Vergleich zum ppp-GFP2-Duplex und zum direkt über eine Phosphodiesterbindung 5'-5'-verknüpften GFP2_s2n55-Dimerduplex in Chloroquin-behandelten PBMCs auf die RIG-I-Aktivität untersucht. Die Konzentrationsangaben beziehen sich auf die Menge an 24mer-Einheiten. Dargestellt sind die Mittelwerte und Standardabweichungen einer Doppelwertbestimmung eines repräsentativen Spenders (n=2).

## Patentansprüche

1. Oligonukleotid-Konjugat K der Struktur
RNA1 -B- RNA2
RNA3 RNA4
oder ein pharmazeutisch verträgliches Salz davon, wobei:
RNA1 einen ersten Strang einer Ribonukleinsäure oder eines Analogons oder Derivats davon von wenigstens sechs Nukleotiden Länge darstellt;
RNA2 einen zweiten Strang einer Ribonukleinsäure oder eines Analogons oder Derivats davon von wenigstens sechs Nukleotiden Länge darstellt;
RNA3 einen dritten Strang einer Ribonukleinsäure oder eines Analogons oder Derivats davon von wenigstens sechs Nukleotiden Länge darstellt, der mindestens fünf komplementäre Basenpaarungen zu RNA1 ausbildet;
RNA4 einen vierten Strang einer Ribonukleinsäure oder eines Analogons oder Derivats davon von wenigstens sechs Nukleotiden Länge darstellt, der mindestens fünf komplementäre Basenpaarungen zu RNA2 ausbildet; und
B einen bivalenten Phosphodiester-Linker mit einem Molekulargewicht von nicht mehr als 1500 Da darstellt, der den 5'-Terminus von RNA1 mit dem 5'-Terminus von RNA2 oder den 3'-Terminus von RNA1 mit dem 3'-Terminus von RNA2 kovalent verbindet,
wobei RNA3 und RNA4 nicht kovalent miteinander verbunden sind,
wobei das Oligonukleotid-Konjugat K ein Aktivator des zytosolischen Helikase Retinsäure-Induzierbaren Gens I (RIG-I) ist,, und
wobei RNA1 und RNA3, sowie RNA2 und RNA4 jeweils keinen Überhang der 5'-terminalen Nukleotidreste und nicht mehr als fünf Nukleotide Überhang der 3'-terminalen Nukleotidreste aufweisen,
zur Verwendung in einem Verfahren zum Behandeln oder Vorbeugen eines Tumors und/oder einer viralen Infektion.

2. Oligonukleotid-Konjugat K zur Verwendung gemäß Anspruch 1, wobei die Sequenz von RNA2 der Sequenz von RNA1 identisch ist.

3. Oligonukleotid-Konjugat K zur Verwendung gemäß einem der Ansprüche 1 oder 2, wobei die Sequenz von RNA3 der Sequenz von RNA4 identisch ist.

4. Oligonukleotid-Konjugat K zur Verwendung gemäß einem der Ansprüche 1 bis 3, wobei B einen bivalenten Linker darstellt, der
den 5'-Terminus von RNA1 mit dem 5'-Terminus von RNA2 kovalent verbindet, und wobei RNA3 und RNA4 an ihren 5'-Termini jeweils Triphosphatreste, Triphosphatanalogonreste oder freie Hydroxylgruppen, bevorzugt jeweils Triphosphatreste oder freie Hydroxylgruppen, insbesondere jeweils Triphosphatreste aufweisen; oder
den 3'-Terminus von RNA1 mit dem 3'-Terminus von RNA2 kovalent verbindet, und wobei RNA1 und RNA2 an ihren 5'-Termini jeweils Triphosphatreste, Triphosphatanalogonreste oder freie Hydroxylgruppen, bevorzugt jeweils Triphosphatreste oder freie Hydroxylgruppen, insbesondere jeweils Triphosphatreste aufweisen.

5. Oligonukleotid-Konjugat K zur Verwendung gemäß einem der Ansprüche 1 bis 4, wobei RNA1, RNA2, RNA3 und RNA4 jeweils zwischen 10 und 50, bevorzugt zwischen 15 und 40, stärker bevorzugt zwischen 19 und 30, insbesondere zwischen 20 und 25 Nukleotide Länge aufweisen.

6. Oligonukleotid-Konjugat K zur Verwendung gemäß einem der Ansprüche 1 bis 5, wobei RNA1 und RNA3, sowie RNA2 und RNA4 jeweils keinen Überhang der 5'-terminalen Nukleotidreste und nicht mehr als vier, stärker bevorzugt nicht mehr als drei, noch stärker bevorzugt nicht mehr als zwei, noch stärker bevorzugt nicht mehr als ein Nukleotid Überhang der 3'-terminalen Nukleotidreste aufweisen, insbesondere keinen Unterschied in der Länge des Nukleotidstrangs aufweisen, insbesondere wobei RNA1 zu RNA3 vollständig komplementär ist und RNA2 zu RNA4 vollständig komplementär ist.

7. Oligonukleotid-Konjugat K zur Verwendung gemäß einem der Ansprüche 1 bis 6, wobei die Sequenz von RNA2 mit der Sequenz von RNA1 identisch ist und die Sequenz von RNA3 der Sequenz von RNA4 identisch ist,
die Stränge RNA1, RNA2, RNA3, RNA4 jeweils gleich lang sind,
RNA1 zu RNA3 vollständig komplementär ist und
RNA2 zu RNA4 vollständig komplementär ist.

8. Oligonukleotid-Konjugat K zur Verwendung gemäß einem der Ansprüche 1 bis 7, wobei:
RNA1 oder RNA3 eine Sequenzhomologie zu SEQ ID NO: 1 von mindestens 80% aufweist, insbesondere wobei RNA1 und RNA2 oder RNA3 und RNA4 jeweils eine Sequenzhomologie zu SEQ ID NO: 1 von mindestens 80% aufweisen; und/oder
RNA1 und RNA2 oder RNA3 und RNA4 jeweils eine Sequenzhomologie zu SEQ ID NO: 1 von mindestens 80% aufweisen und RNA3 und RNA4 oder RNA1 und RNA2 jeweils eine Sequenzhomologie zu SEQ ID NO: 2 von mindestens 80% aufweisen.

9. Pharmazeutische Zusammensetzung zur Verwendung in einem Verfahren zum Behandeln oder Vorbeugen eines Tumors und/oder einer viralen Infektion, umfassend ein Oligonukleotid-Konjugat K zur Verwendung gemäß einem der Ansprüche 1 bis 8 und einen pharmazeutisch verträglichen Träger.

10. Oligonukleotid-Konjugat K zur Verwendung gemäß einem der Ansprüche 1 bis 8 oder pharmazeutische Zusammensetzung zur Verwendung gemäß Anspruch 9, wobei das Oligonukleotid-Konjugat K in Kombination mit einem oder mehreren antiproliferativen oder antiviralen Wirkstoffen eingesetzt wird.

11. Verwendung eines Oligonukleotid-Konjugats K der Struktur
RNA1 -B- RNA2
RNA3 RNA4
oder ein pharmazeutisch verträgliches Salz davon, wobei:
RNA1 einen ersten Strang einer Ribonukleinsäure oder eines Analogons oder Derivats davon von wenigstens sechs Nukleotiden Länge darstellt;
RNA2 einen zweiten Strang einer Ribonukleinsäure oder eines Analogons oder Derivats davon von wenigstens sechs Nukleotiden Länge darstellt;
RNA3 einen dritten Strang einer Ribonukleinsäure oder eines Analogons oder Derivats davon von wenigstens sechs Nukleotiden Länge darstellt, der mindestens fünf komplementäre Basenpaarungen zu RNA1 ausbildet;
RNA4 einen vierten Strang einer Ribonukleinsäure oder eines Analogons oder Derivats davon von wenigstens sechs Nukleotiden Länge darstellt, der mindestens fünf komplementäre Basenpaarungen zu RNA2 ausbildet; und
B einen bivalenten Phosphodiester-Linker mit einem Molekulargewicht von nicht mehr als 1500 Da darstellt, der den 5'-Terminus von RNA1 mit dem 5'-Terminus von RNA2 oder den 3'-Terminus von RNA1 mit dem 3'-Terminus von RNA2 kovalent verbindet,
wobei RNA3 und RNA4 nicht kovalent miteinander verbunden sind,
wobei das Oligonukleotid-Konjugat K ein Aktivator des zytosolischen Helikase Retinsäure-Induzierbaren Gens I (RIG-I) ist, und
wobei RNA1 und RNA3, sowie RNA2 und RNA4 jeweils keinen Überhang der 5'-terminalen Nukleotidreste und nicht mehr als fünf Nukleotide Überhang der 3'-terminalen Nukleotidreste aufweisen,
zur Induktion vermehrter Sezernierung von Interferon-alpha *in vitro.*

## Claims

1. Oligonucleotide conjugate K of the structure RNA1 -B- RNA2 RNA3 RNA4 or a pharmaceutically compatible salt thereof, wherein:
RNA1 represents a first strand of a ribonucleic acid or an analogue or derivative thereof of at least six nucleotides in length;
RNA2 represents a second strand of a ribonucleic acid or an analogue or derivative thereof of at least six nucleotides in length;
RNA3 represents a third strand of a ribonucleic acid or an analogue or derivative thereof of at least six nucleotides in length, which forms at least five complementary base pairs with RNA1;
RNA4 represents a fourth strand of a ribonucleic acid or an analogue or derivative thereof of at least six nucleotides in length, which forms at least five complementary base pairs with RNA2; and
B represents a bivalent phosphodiester linker having a molecular weight of not more than 1500 Da that covalently bonds the 5' terminus of RNA1 to the 5' terminus of RNA2 or the 3' terminus of RNA1 to the 3' terminus of RNA2,
wherein RNA3 and RNA4 are not covalently bonded to one another,
wherein the oligonucleotide conjugate K is an activator of the cytosolic helicase retinoic acid-inducible gene I (RIG-I), and
wherein RNA1 and RNA3, and RNA2 and RNA4, each have no overhang of the 5'-terminal nucleotide residues and an overhang of the 3'-terminal nucleotide residues of not more than five nucleotides,
for use in a method of treating or precluding a tumour and/or a viral infection.

2. Oligonucleotide conjugate K for use according to Claim 1, wherein the sequence of RNA2 is identical to the sequence of RNA1.

3. Oligonucleotide conjugate K for use according to either of Claims 1 and 2, wherein the sequence of RNA3 is identical to the sequence of RNA4.

4. Oligonucleotide conjugate K for use according to any of Claims 1 to 3, wherein B represents a bivalent linker that
covalently bonds the 5' terminus of RNA1 to the 5' terminus of RNA2, and wherein RNA3 and RNA4 each have, at their 5' termini, triphosphate residues, triphosphate analogue residues or free hydroxyl groups, preferably each have triphosphate residues or free hydroxyl groups, and especially each have triphosphate residues; or
covalently bonds the 3' terminus of RNA1 to the 3' terminus of RNA2, and wherein RNA1 and RNA2 each have, at their 5' termini, triphosphate residues, triphosphate analogue residues or free hydroxyl groups, preferably each have triphosphate residues or free hydroxyl groups, and especially each have triphosphate residues.

5. Oligonucleotide conjugate K for use according to any of Claims 1 to 4, wherein RNA1, RNA2, RNA3 and RNA4 each have a length of between 10 and 50, preferably between 15 and 40, more preferably between 19 and 30 and especially between 20 and 25 nucleotides.

6. Oligonucleotide conjugate K for use according to any of Claims 1 to 5, wherein RNA1 and RNA3, and RNA2 and RNA4, each have no overhang of the 5'-terminal nucleotide residues and an overhang of the 3'-terminal nucleotide residues of not more than four nucleotides, more preferably not more than three nucleotides, even more preferably not more than two nucleotides and even more preferably not more than one nucleotide, especially no difference in the length of the nucleotide strand, especially wherein RNA1 is fully complementary to RNA3 and RNA2 is fully complementary to RNA4.

7. Oligonucleotide conjugate K for use according to any of Claims 1 to 6, wherein the sequence of RNA2 is identical to the sequence of RNA1 and the sequence of RNA3 is identical to the sequence of RNA4,
the strands RNA1, RNA2, RNA3, RNA4 are each of equal length,
RNA1 is fully complementary to RNA3 and
RNA2 is fully complementary to RNA4.

8. Oligonucleotide conjugate K for use according to any of Claims 1 to 7, wherein:
RNA1 or RNA3 have a sequence homology to SEQ ID NO: 1 of at least 80%, especially wherein RNA1 and RNA2 or RNA3 and RNA4 each have a sequence homology to SEQ ID NO: 1 of at least 80%; and/or
RNA1 and RNA2 or RNA3 and RNA4 each have a sequence homology to SEQ ID NO: 1 of at least 80% and RNA3 and RNA4 or RNA1 and RNA2 each have a sequence homology to SEQ ID NO: 2 of at least 80%.

9. Pharmaceutical composition for use in a method of treating or precluding a tumour and/or a viral infection, comprising an oligonucleotide conjugate K for use according to any of Claims 1 to 8 and a pharmaceutically acceptable vehicle.

10. Oligonucleotide conjugate K for use according to any of Claims 1 to 8 or pharmaceutical composition for use according to Claim 9, wherein the oligonucleotide conjugate K is used in combination with one or more active antiproliferative or antiviral ingredients.

11. Use of an oligonucleotide conjugate K of the structure
RNA1 -B- RNA2
RNA3 RNA4
or a pharmaceutically compatible salt thereof, wherein:
RNA1 represents a first strand of a ribonucleic acid or an analogue or derivative thereof of at least six nucleotides in length;
RNA2 represents a second strand of a ribonucleic acid or an analogue or derivative thereof of at least six nucleotides in length;
RNA3 represents a third strand of a ribonucleic acid or an analogue or derivative thereof of at least six nucleotides in length, which forms at least five complementary base pairs with RNA1;
RNA4 represents a fourth strand of a ribonucleic acid or an analogue or derivative thereof of at least six nucleotides in length, which forms at least five complementary base pairs with RNA2; and
B represents a bivalent phosphodiester linker having a molecular weight of not more than 1500 Da that covalently bonds the 5' terminus of RNA1 to the 5' terminus of RNA2 or the 3' terminus of RNA1 to the 3' terminus of RNA2,
wherein RNA3 and RNA4 are not covalently bonded to one another,
wherein the oligonucleotide conjugate K is an activator of the cytosolic helicase retinoic acid-inducible gene I (RIG-I), and
wherein RNA1 and RNA3, and RNA2 and RNA4, each have no overhang of the 5'-terminal nucleotide residues and an overhang of the 3'-terminal nucleotide residues of not more than five nucleotides,
for induction of increased secretion of interferon alpha *in vitro.*

## Revendications

1. Conjugué oligonucléotidique K de structure
ARN1 -B- ARN2
ARN3 ARN4
ou un sel pharmaceutiquement acceptable de celui-ci, où :
ARN1 représente un premier brin d'un acide ribonucléique ou d'un analogue ou d'un dérivé de celui-ci d'une longueur d'au moins six nucléotides ;
ARN2 représente un deuxième brin d'un acide ribonucléique ou d'un analogue ou d'un dérivé de celui-ci d'une longueur d'au moins six nucléotides ;
ARN3 représente un troisième brin d'un acide ribonucléique ou d'un analogue ou d'un dérivé de celui-ci d'une longueur d'au moins six nucléotides, qui forment au moins cinq paires de bases complémentaires à ARN1 ;
ARN4 représente un quatrième brin d'un acide ribonucléique ou d'un analogue ou d'un dérivé de celui-ci d'une longueur d'au moins six nucléotides, qui forment au moins cinq paires de bases complémentaires à ARN2 ;
B représente un lieur phosphodiester divalent présentant un poids moléculaire qui n'est pas supérieur à 1500 Da, qui relie par covalence l'extrémité 5' d'ARN1 à l'extrémité 5' d'ARN2 ou l'extrémité 3' d'ARN1 à l'extrémité 3' d'ARN2,
ARN3 et ARN4 n'étant pas reliés par covalence l'un à l'autre,
le conjugué oligonucléotidique K étant un activateur de l'hélicase cytosolique qui est le gène I inductible par l'acide rétinoïque (RIG-I) et
ARN1 et ARN3 ainsi que ARN2 et ARN4 ne présentant respectivement pas de débordement des restes nucléotidiques en l'extrémité 5' et ne présentant pas plus de cinq nucléotides de débordement des restes nucléotidiques en l'extrémité 3',
pour une utilisation dans un procédé pour le traitement ou la prévention d'une tumeur et/ou d'une infection virale.

2. Conjugué oligonucléotidique K pour une utilisation selon la revendication 1, la séquence d'ARN2 étant identique à la séquence d'ARN1.

3. Conjugué oligonucléotidique K pour une utilisation selon l'une quelconque des revendications 1 ou 2, la séquence d'ARN3 étant identique à la séquence d'ARN4.

4. Conjugué oligonucléotidique K pour une utilisation selon l'une quelconque des revendications 1 à 3, B étant un lieur divalent qui
- relie par covalence l'extrémité 5' d'ARN1 à l'extrémité 5' d'ARN2, ARN3 et ARN4 présentant au niveau de leurs extrémités 5' à chaque fois des radicaux triphosphate, des radicaux d'analogue de triphosphate ou des groupes hydroxyle libres, de préférence à chaque fois des radicaux triphosphate ou des groupes hydroxyle libres, en particulier à chaque fois des radicaux triphosphate ; ou
- relie par covalence l'extrémité 3' d'ARN1 à l'extrémité 3' d'ARN2, ARN1 et ARN2 présentant au niveau de leurs extrémités 5' à chaque fois des radicaux triphosphate, des radicaux d'analogue de triphosphate ou des groupes hydroxyle libres, de préférence à chaque fois des radicaux triphosphate ou des groupes hydroxyle libres, en particulier à chaque fois des radicaux triphosphate.

5. Conjugué oligonucléotidique K pour une utilisation selon l'une quelconque des revendications 1 à 4, ARN1, ARN2, ARN3 et ARN4 présentant à chaque fois une longueur d'entre 10 et 50, de préférence d'entre 15 et 40, plus préférablement d'entre 19 et 30, en particulier d'entre 20 et 25 nucléotides.

6. Conjugué oligonucléotidique K pour une utilisation selon l'une quelconque des revendications 1 à 5, ARN1 et ARN3 ainsi que ARN2 et ARN4 ne présentant pas de débordement des restes nucléotidiques en l'extrémité 5' et ne présentant pas plus de quatre nucléotides, plus préférablement pas plus de trois nucléotides, encore plus préférablement pas plus de deux nucléotides, encore plus préférablement pas plus d'un nucléotide de débordement des restes nucléotidiques en l'extrémité 3' et ne présentant en particulier pas de différence dans la longueur du brin nucléotidique, en particulier ARN1 étant totalement complémentaire à ARN3 et ARN2 étant totalement complémentaire à ARN4.

7. Conjugué oligonucléotidique K pour une utilisation selon l'une quelconque des revendications 1 à 6,
la séquence d'ARN2 étant identique à la séquence d'ARN1 et la séquence d'ARN3 étant identique à la séquence d'ARN4,
les brins d'ARN1, d'ARN2, d'ARN3, d'ARN4 présentant à chaque fois la même longueur,
ARN1 étant totalement complémentaire à ARN3 et
ARN2 étant totalement complémentaire à ARN4.

8. Conjugué oligonucléotidique K pour une utilisation selon l'une quelconque des revendications 1 à 7,
- ARN1 ou ARN3 présentant une homologie de séquence avec la séquence SEQ ID NO : 1 d'au moins 80%, en particulier ARN1 et ARN2 ou ARN3 et ARN4 présentant à chaque fois une homologie de séquence avec la séquence SEQ ID NO : 1 d'au moins 80% ; et/ou
- ARN1 et ARN2 ou ARN3 et ARN4 présentant à chaque fois une homologie de séquence avec la séquence SEQ ID NO : 1 d'au moins 80% et ARN3 et ARN4 ou ARN1 et ARN2 présentant à chaque fois une homologie de séquence avec la séquence SEQ ID NO : 2 d'au moins 80%.

9. Composition pharmaceutique pour une utilisation dans un procédé pour le traitement ou la prévention d'une tumeur et/ou d'une infection virale, comprenant un conjugué oligonucléotidique K pour une utilisation selon l'une quelconque des revendications 1 à 8 et un support pharmaceutiquement acceptable.

10. Conjugué oligonucléotidique K pour une utilisation selon l'une quelconque des revendications 1 à 8 ou composition pharmaceutique pour une utilisation selon la revendication 9, le conjugué oligonucléotidique K étant utilisé en combinaison avec un ou plusieurs principes actifs antiprolifératifs ou antiviraux.

11. Utilisation d'un conjugué oligonucléotidique K de structure
ARN1 -B- ARN2
ARN3 ARN4
ou d'un sel pharmaceutiquement acceptable de celui-ci, où :
ARN1 représente un premier brin d'un acide ribonucléique ou d'un analogue ou d'un dérivé de celui-ci d'une longueur d'au moins six nucléotides ;
ARN2 représente un deuxième brin d'un acide ribonucléique ou d'un analogue ou d'un dérivé de celui-ci d'une longueur d'au moins six nucléotides ;
ARN3 représente un troisième brin d'un acide ribonucléique ou d'un analogue ou d'un dérivé de celui-ci d'une longueur d'au moins six nucléotides, qui forment au moins cinq paires de bases complémentaires à ARN1 ;
ARN4 représente un quatrième brin d'un acide ribonucléique ou d'un analogue ou d'un dérivé de celui-ci d'une longueur d'au moins six nucléotides, qui forment au moins cinq paires de bases complémentaires à ARN2 ;
B représente un lieur phosphodiester divalent présentant un poids moléculaire qui n'est pas supérieur à 1500 Da, qui relie par covalence l'extrémité 5' d'ARN1 à l'extrémité 5' d'ARN2 ou l'extrémité 3' d'ARN1 à l'extrémité 3' d'ARN2,
ARN3 et ARN4 n'étant pas reliés par covalence l'un à l'autre,
le conjugué oligonucléotidique K étant un activateur de l'hélicase cytosolique qui est le gène I inductible par l'acide rétinoïque (RIG-I) et
ARN1 et ARN3 ainsi que ARN2 et ARN4 ne présentant respectivement pas de débordement des restes nucléotidiques en l'extrémité 5' et ne présentant pas plus de cinq nucléotides de débordement des restes nucléotidiques en l'extrémité 3',
pour l'induction d'une sécrétion augmentée d'interféron alpha in vitro.


### SEQUENZPROTOKOLL

<110> Rheinische Friedrich-Wilhelms-Universität Bonn
<120> Diskontinuierliche Oligonukleotid-Liganden
<130> P69764PC
<160> 3
<170> PatentIn version 3.5
<210> 1
   <211> 24
   <212> RNA
   <213> artifiziell
<400> 1
   gacgcugacc cugaaguuca ucuu 24
<210> 2
   <211> 24
   <212> RNA
   <213> artifiziell
<400> 2
   aagaugaacu ucagggucag cguc 24
<210> 3
   <211> 48
   <212> RNA
   <213> artifiziell
<400> 3
   gacgcugacc cugaaguuca ucuugacgcu gacccugaag uucaucuu 48